# EUROPEAN PATENT APPLICATION

(11) **EP 4 545 092 A1**
(43) Date of publication of application: **30.04.2025**
(21) Application number: 23206487.3
(22) Date of filing: 27.10.2023
(51) Int. Cl.: A61K 39/00

(54) **COMPUTATIONALLY DESIGNED BINDERS FOR IMMUNE CELL THERAPIES**

(71) Applicant: Technische Universität München, 80333 München (DE)
(72) Inventor: The designation of the inventor has not yet been filed
(74) Representative: Engelhard, Markus

(57) **Abstract**

The invention is based on an immunomodulatory molecule, comprising an engineered protein binding domain, a linker and/or a hinge-region, and a domain for the activation and/or stimulation of an immune effector cell. The invention also pertains to an isolated nucleic acid comprising a sequence encoding for an immunomodulatory molecule according to this invention. Further provided is a method of obtaining a chimeric antigen receptor (CAR) expressing immune effector cell product, such as a CAR-T cell product, and/or a HLA-independent T cell receptor (HIT) expressing immune effector cell product, such as a HIT-T cell product, and/or a bispecific molecule expressing immune effector cell product, such as a bispecific T cell engager (BiTE) expressing immune effector cell product, comprising an immunomodulatory molecule, and/or a bispecific molecule, such as a BiTE, comprising an engineered protein binding domain. The invention also pertains to a product thereby obtainable, and to a pharmaceutical composition comprising the immunomodulatory molecule, the isolated nucleic acid, or the product obtainable by a method according to the present invention. Also provided is an immunomodulatory molecule, an isolated nucleic acid, a product, or a pharmaceutical composition according to this invention, for use in a method of diagnosis, prevention and/or treatment of a disease, or for preventing an organ transplant rejection, in a patient in need thereof.

## Description

### FIELD OF THE INVENTION

The invention is based on an immunomodulatory molecule, comprising an engineered protein binding domain, a linker and/or a hinge-region, and a domain for the activation and/or stimulation of an immune effector cell. The invention also pertains to an isolated nucleic acid comprising a sequence encoding for an immunomodulatory molecule according to this invention. Further provided is a method of obtaining a chimeric antigen receptor (CAR) expressing immune effector cell product, such as a CAR-T cell product, and/or a HLA-independent T cell receptor (HIT) expressing immune effector cell product, such as a HIT-T cell product, and/or a bispecific molecule expressing immune effector cell product, such as a bispecific T cell engager (BiTE) expressing immune effector cell product, comprising an immunomodulatory molecule, and/or a bispecific molecule, such as a BiTE, comprising an engineered protein binding domain. The invention also pertains to a product thereby obtainable, and to a pharmaceutical composition comprising the immunomodulatory molecule, the isolated nucleic acid, or the product obtainable by a method according to the present invention. Also provided is an immunomodulatory molecule, an isolated nucleic acid, a product, or a pharmaceutical composition according to this invention, for use in a method of diagnosis, prevention and/or treatment of a disease, or for preventing an organ transplant rejection, in a patient in need thereof.

### DESCRIPTION

Recent progress in the field indicated that CAR-T cells and BiTEs can be used for the treatment of different kinds of diseases, such as cancer. CARs and BiTEs provide a way to direct a T cell effector response, e.g., cytotoxicity, to target cells expressing a selected target antigen, often a tumor antigen or tissue-specific antigen. CARs are an adaptation of the T cell receptor, where the antigen binding domain is replaced with the antigen binding domain of an antibody that specifically binds the derived target antigen.

BiTEs are polypeptides that each include tandemly linked single-chain variable fragments (scFv). Optionally, the scFvs are connected by a linker. One scFv of the BiTE binds to the T cell receptor (e.g., to the CD₃ epsilon subunit), and the other binds to a target antigen. Engagement of the target antigen on the surface of a target cell by a CAR expressed on a T cell ('CAR-T cell') or by a BiTE promotes effector function, e.g., the killing of the target cell.

CAR-T cells have outstanding clinical success in treating B cell leukemias and lymphomas. All six currently approved CAR-T cell products employ antigen-binding domains derived from therapeutic antibodies (monoclonal antibodies, heavy-chain-only antibodies). However, traditional hybridoma-based monoclonal antibody production against a target of interest is very tedious and expensive. Moreover, such traditional monoclonal antibody production techniques are further limited since proteins that are highly conserved across species can be challenging targets, and small peptides often need to be conjugated to carrier proteins. The process of CAR-T development is currently also slow and expensive.

scFv-derived CARs typically have a very high affinity, yet it is currently unclear how this impacts the long-term performance of the corresponding CAR-T. Preclinically, other binding domains have successfully been employed for CAR design, such as either naturally occurring receptor-ligand pairs (e.g. BCMA/TACI - APRIL, CD₇₀ - CD₂₇, IL₁₃Ra₂ - zetakine) or antibody mimetics, like DARPins or affibody molecules. DARPins and affibody molecules have both been designed based on naturally occurring proteins (ankyrin proteins and Protein A from Staphylococcus aureus, respectively). In both cases, antibody mimetics with desired binding properties for a specific target are identified by phage display or ribosome display technology from a vast library of candidates.

However, despite much progress in the field, critical challenges still need to be addressed to accelerate the clinical translation of these therapies. The development of therapeutic antibodies and/or antibody-based binders is extremely time-consuming, complicated, and cost and resource intensive (requiring, e.g., a highly specialized infrastructure and complex animal models). Specifically, the development of a single antibody-based binder often requires a multi-year process for its generation. Subsequently, a phage display and/or other validations are often required.

US 6217866 B1 discloses a non-computational method to generate monoclonal antibodies that target EGFR, and therapeutic uses thereof.

Cao, L. *et al.* discloses a computational method to design protein-binding proteins from a target structure.^{[1]}

Bennett, N. *et al.* discloses a method for improving de novo Protein Binder Design with Deep Learning.^{[2]}

Watson, J. *et al.* discloses a method for improving de novo Protein Binder Design with Diffusion Models.^{[3]}

To help solve some of the current challenges, the inventors sought to test the feasibility of employing computational and generative target-to-binder models for rapid and straightforward design of de novo protein binders, using the target protein sequence and structure as the primary input, and incorporating these de novo protein binders into CARs, HITs, and/or BiTEs.

Thus, it is an object of the invention to provide a fast, simple and cheap way to generate immunomodulatory molecules comprising an engineered protein binding domain, and to provide engineered CAR-T cells, HITs, and/or BiTEs with customizable properties. It is a further object to provide immunomodulatory molecules comprising an engineered protein binding domain having a reduced immunogenicity compared to conventionally used immunomodulatory molecules. Yet another object is to provide immunomodulatory molecules comprising an engineered protein binding domain that are less likely to induce patient immune responses compared to scFvs derived from antibodies raised in animals or bacterial cell systems (such as by phage display). It is a further object to provide immunomodulatory molecules comprising an engineered protein binding domain that are suited for ex vivo manufacturing of immune therapies and in vivo delivery into immune cells. It is a further object to make use of computational and generative target-to-binder models to provide a fast, easy and cheap way to generate immunomodulatory molecules comprising an engineered protein binding domain. It is a further object to generate immunomodulatory molecules comprising an engineered protein binding domain targeting neo-epitopes and/or having new properties, optionally wherein said new properties make them suitable for the use in the treatment of a rare diseases.

### BRIEF DESCRIPTION OF THE INVENTION

Generally, and by way of brief description, the main aspects of the present invention can be described as follows:

In a first aspect, the invention pertains to an immunomodulatory molecule, comprising an engineered protein binding domain, a linker and/or a hinge-region, and a domain for the activation and/or stimulation of an immune effector cell.

In a second aspect, the invention pertains to an engineered protein binding domain, wherein said engineered protein binding domain is removed from an immunomodulatory molecule according to the first aspect of this invention, and wherein said engineered protein binding domain is used outside of the immunomodulatory molecule, optionally wherein said engineered protein binding domain is fused to an Fc-part or a CH₃ domain of an antibody, and/or and wherein said engineered protein binding domain is used as a binder drug conjugate.

In a third aspect, the invention pertains to an isolated nucleic acid comprising a sequence encoding for an immunomodulatory molecule according to the first aspect of this invention, or encoding for an engineered protein binding domain according to the second aspect of this invention, or an expression construct for expressing the immunomodulatory molecule according to the first aspect of this invention in a (host) cell, preferably further comprising a promoter and/or a terminator sequence.

In a fourth aspect, the invention pertains to a method of obtaining a chimeric antigen receptor (CAR) expressing immune effector cell product, such as a CAR-T cell product, and/or a HLA-independent T cell receptor (HIT) expressing immune effector cell product, such as a HIT-T cell product, and/or a bispecific molecule expressing immune effector cell product, such as a bispecific T cell engager (BiTE) expressing immune effector cell product, comprising an immunomodulatory molecule, and/or a bispecific molecule, such as a bispecific T cell engager (BiTE), comprising an engineered protein binding domain.

In a fifth aspect, the invention pertains to a product obtainable by a method according to the fourth aspect of this invention.

In a sixth aspect, the invention pertains to an immunomodulatory molecule according to the first aspect of this invention, an engineered protein binding domain according to the second aspect of this invention, the isolated nucleic acid according to the third aspect of this invention, or the product according to the fifth aspect of the present invention, wherein the immunomodulatory molecule is a CAR.

In a seventh aspect, the invention pertains to a pharmaceutical composition comprising the immunomodulatory molecule according to the first aspect of this invention, an engineered protein binding domain according to the second aspect of this invention, the isolated nucleic acid according to the third aspect of this invention, the product according to the fifth aspect of the present invention, or the immunomodulatory molecule, the isolated nucleic acid, or the product according to the sixth aspect of the present invention.

In an eighth aspect, the invention pertains to the immunomodulatory molecule according to the first aspect of this invention, the engineered protein binding domain according to the second aspect of this invention, the isolated nucleic acid according to the third aspect of this invention, the product according to the fifth aspect of this invention, the immunomodulatory molecule, the isolated nucleic acid, or the product according to the sixth aspect of the present invention, or the pharmaceutical composition according to the seventh aspect of this invention, for use in a method of diagnosis, prevention and/or treatment of a disease, or for preventing an organ transplant rejection, in a patient in need thereof.

In a ninth aspect, the invention pertains to a method of treatment of a patient using a host cell comprising a coding sequence encoding the immunomodulatory molecule according to the first aspect of this invention, or comprising a coding sequence encoding the engineered protein binding domain according to the second aspect of this invention, or the isolated nucleic acid according to the third aspect of this invention.

In a tenth aspect, the present invention pertains to a T cell, such as a CAR-T cell, comprising a coding sequence encoding an immunomodulatory molecule according to the first aspect of this invention, or a product obtainable by a method according to the fourth aspect of this invention.

In an eleventh aspect, the present invention pertains to a kit.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

Before disclosing the subject-matter in greater detail, definitions of terms/expressions as used herein are provided.

Unless otherwise defined, all technical and scientific terms used herein have the same meaning as commonly understood by one of ordinary skill in the art to which this invention belongs. Although methods and materials similar to those described herein can be used in the practice or testing of the present invention, suitable methods and materials are described below. In addition, the materials, methods, and examples are illustrative only and not intended to be limiting.

Each of the patents, applications and articles cited herein, and each document cited or referenced therein, including during the prosecution of any of the patents and/or applications cited herein ("patent cited documents"), and any manufacturer's instructions or catalogues for any products cited herein or mentioned in any of the references and in any of the patent cited documents, are hereby incorporated herein by reference. Documents incorporated by reference into this text or any teachings therein may be used in the practice of this invention. Documents incorporated by reference into this text are not admitted to be prior art. As used herein, the words "may" and "maybe" are to be interpreted in an open-ended, non-restrictive manner. At minimum, "may" and "may be" are to be interpreted as definitively including structure or acts recited.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

The term "immunomodulatory molecule", as used herein, refers to a molecule that is capable of modulating the activity of an immune system upon binding of the immunomodulatory molecule to a target molecule. In one embodiment, the term "immunomodulatory molecule" refers to a molecule that is capable of enhancing or diminishing the activity of an immune system upon binding of the immunomodulatory molecule to a target molecule. An immunomodulatory molecule may also be referred to as "modulator of immune effector cells" or "activator of immune effector cells". An immunomodulatory molecule may also be referred to as "immune stimulating molecule" or a "protein binding molecule". In some embodiments, the immunomodulatory molecule is a chimeric antigen receptor (CAR). In such embodiments, the immunomodulatory molecule according to the present invention can also be referred to as "compCAR" (referring to computationally designed binder/ computationally designed CAR). In some embodiments, the immunomodulatory molecule according to the present invention is a bispecific molecule, such as a bispecific T cell engager (BiTE). In some embodiments, the immunomodulatory molecule according to the present invention is a HLA-independent T cell receptor (HIT).

As used herein, the term "engineered protein binding domain" shall refer to a protein binding domain that has been newly designed, i.e. a de novo designed protein binding domain. The engineered protein binding domains according to this invention shall include protein binding domains generated with computational methods, e.g., using artificial intelligence (AI). The term "engineered protein binding domain" according to the present invention can also be referred to as "computationally designed protein binding domain", "computationally designed binder" and as "AI-designed protein binding domain". As such, the terms "engineered protein binding domain", "computationally designed protein binding domain", "computationally designed binder", and "AI-designed protein binding domain" can be used interchangeably.

The term "protein binding domain" can also be referred to as "antigen binding domain", "antigen binding moiety", and as "target binding domain". Accordingly, the term "engineered protein binding domain", as used herein, shall also refer to an "engineered antigen binding domain", an "engineered antigen binding moiety", and an "engineered target binding domain". The term "engineered protein binding domain", as used herein, shall further refer to a "computationally designed antigen binding domain", a "computationally designed antigen binding moiety", and a "computationally designed target binding domain".

The term "DARPin", as used herein, is an acronym for "designed ankyrin repeat proteins". The term "DARPin" shall refer to a small, single domain protein of around 14 kDa, which is capable of binding antigens. DARPins are genetically engineered antibody mimetic proteins typically exhibiting highly specific and high-affinity target protein binding. DARPins are derived from natural ankyrin repeat proteins that consist of at least three repeat motifs or modules.

The term "ribosome/phage display techniques", as used herein, refers to an in vitro screening technique for identifying ligands for proteins and other macromolecules. In the "phage display technique", also known as "phage display", a gene encoding a protein of interest is inserted into a gene encoding a phage coat protein. This causes a phage to "display" the protein on its outside. Proteins displayed by the phages can then be screened against other proteins and macromolecules, to detect interactions between the displayed protein and those other molecules. The term "ribosome display" refers to a cell-free system for the in vitro selection of proteins and peptides from large libraries. In this system, stable protein-ribosome-mRNA (PRM) complexes are allowed to bind to a surface-bound ligand. Complexes that bind well are immobilized and mRNA of the complexes displaying a binding polypeptide can be recovered, and thus, the genetic information of the binding polypeptides is available for analysis.

The term "affibody", as used herein, shall refer to small engineered proteins that imitate monoclonal antibodies and are able to bind antigens. Affibodies, also called affibody molecules, consist of three alpha helices with 58 amino acids and have a molar mass of about 6 kDa.

The term "binding" according to the present invention shall refer to a specific binding to a target region and/or a target antigen. The terms "binding" and "specifically binding" can be used interchangeably according to the present invention.

In the context of the present invention, the term "antigen", as used herein, shall refer to a peptide or a protein which may be recognized by the immune system, preferably by the adaptive immune system. An antigen is usually capable of triggering an antigen-specific immune response, e.g. by formation of antibodies and/or antigen-specific T cells as part of an adaptive immune response. An antigen usually comprises at least one epitope, which may be presented by MHC to T cells. In the context of the present invention, also fragments, variants and derivatives of peptides and proteins comprising at least one epitope shall be understood as antigens.

The term "binding kinetic", as used herein, shall refer to a measurement of how fast a compound binds to its target and how fast it dissociates from the target. The term "binding kinetic" shall refer to an on-rate and an off-rate. The person of skill is well aware of ways for measuring a binding , such as by surface plasmon resonance sensograms of the binding between the binding domain linked to single-chain constant fragments (binding-domain-Fcs) and the target antigen.

The terms "binding affinity" and "binding avidity", as used herein, describe the binding strength between an immunomodulatory molecule and antigen. The term "binding affinity" shall refer to the strength of a single bond or interaction, i.e. to the strength of the interaction between the antigen's epitope and the paratope of the immunomodulatory molecule at a singular binding site. A binding affinity can be described as the total of all forces that result in increased binding strength (Kon) minus all of the forces that result in decreased binding strength (Koff). The person of skill is well aware of ways for measuring the binding affinity between an immunomodulatory molecule according to this invention and the corresponding antigen, such as by surface plasmon resonance sensograms of the binding between the binding domain linked to single-chain constant fragments (binding-domain-Fcs) and the target antigen.

The term "binding avidity" shall refer to the accumulated strength of multiple affinities of individual non-covalent binding interactions, such as between an immunomodulatory molecule according to this invention and the corresponding antigen. Avidity differs from affinity, which describes the strength of a single interaction. However, the term "avidity" usually does not correspond to the mere sum of its constituent affinities but as the combined effect of all affinities participating in the biomolecular interaction, because individual binding events increase the likelihood of occurrence of other interactions (i.e., increase the local concentration of each binding partner in proximity to the binding site). The person of skill is well aware of ways for measuring the binding avidity between an immunomodulatory molecule according to this invention and the corresponding antigen, such as by measurement of the immune synapse binding avidity between a cell expressing the immunomodulatory molecule on its surface and a cell expressing the targeted antigen on its surface via acoustic force microfluidic microscopy (e.g. z-Movi assay).

The term "cytotoxicity", as used herein, shall refer to the quality of a compound or an immunomodulatory molecule of being toxic to cells, i.e. of how toxic a compound or an immunomodulatory molecule is to cells. A cytotoxic compound can cause cell damage or death, either through necrosis or apoptosis. The person of skill is well aware of ways for measuring cytotoxicity, such as by measuring ATP content, using protease markers, or using vital dyes such as formazan dyes.

The term "chimeric antigen receptor", or short "CAR", as used herein, refers to a receptor protein that combines both antigen-binding and immune cell activating function. CARs are engineered to give immune effector cells the new ability to target a specific antigen. CARs are also known as chimeric T cell receptors, artificial T cell receptors or chimeric immunoreceptors. CARs comprise a binding domain, such as an engineered binding domain, a transmembrane domain, a hinge-region, an intracellular domain for the activation and/or stimulation of an immune effector cell, such as a T cell. In the context of this disclosure, the binding domain is an engineered binding domain. In CARs, the hinge-region is located between the binding domain and the transmembrane domain. CARs may further comprise a linker, wherein the linker connects the hinge-region and the transmembrane domain. Upon binding of a target antigen, a CAR activates and/or stimulates an immune effector cell via its intracellular domain for the activation and/or stimulation of an immune effector cell. In one embodiment, the intracellular domain for the activation and/or stimulation of an immune effector cell comprises a signaling domain. In a preferred embodiment, the intracellular domain for the activation and/or stimulation of an immune effector cell comprises a signaling domain and one or more co-stimulatory domains.

The term "bispecific molecule", as used herein, refers to a molecule that has at least two different binding specificities. The at least two different binding specificities are conferred by at least two protein binding domains, and the at least two protein binding domains are connected via a hinge-region or a linker. In a preferred embodiment, one of the at least two protein binding domains is a domain for the activation and/or stimulation of an immune effector cell by specifically binding to an immune effector cell and thereby activating and/or stimulating the immune effector cell. In one embodiment, the bispecific molecule is a bispecific T cell engager (BiTE) or has a structure that is comparable to the structure of a BiTE. In the context of this disclosure, the term "bispecific" does not exclude molecules that have more than two specificities. Accordingly, the term "bispecific" also includes an/ or refers to molecules that have three or more specificities, for example three, four, five, six, seven or more binding specificities. In one embodiment, bispecific molecules are produced in cell culture using eukaryotic cells such as HEK293 cells, HEK293T cells, CHO cells, HT-1080 cells, or PER.C6 cells. After production in cell culture, the bispecific molecules can then be purified and applied to a patient in need. In another embodiment, bispecific molecules can be produced by autologous cells, for example in immune effector cells, such as T cells. The use of autologous cells for production of bispecific molecules may have the advantages of better reaching the tumor (e.g. T cells can actively migrate to the tumor, while applied purified only passively spread in blood and tissues). Further endogenous cells can continuously produce the bispecific molecules, thereby ameliorating difficulties in case of short half-life of the molecules. Also, the autologous production of the bispecific molecules enables combination therapies (e.g. CAR-T cells that also produce bispecific molecules, such as BiTEs).

As used herein, the terms "T-cell-engaging antibody", "T-cell-engaging antibodies", "T-cell engager", "bispecific T cell engager" and "BiTE" can be used interchangeably. Accordingly, in any part of this application, the term "bispecific T cell engager" and/or "BiTE" can be exchanged by the term "T-cell-engaging antibody" and/or "T-cell engager". According to this invention, BiTEs can have two scFvs as binding domains ("classical BiTEs"). Additionally, there are also so-called "nanobody BiTEs" with binding domains derived from heavy chain only antibodies.

The term "hinge-region", as used herein, shall refer to a (small) structural protein domain that is connected to the engineered protein binding domain. According to this invention, when the immunomodulatory molecule is a CAR, the hinge-region is located between the engineered protein binding domain and the transmembrane domain. Moreover, according to this invention, when the immunomodulatory molecule is a CAR, the hinge-region is either directly connected to the transmembrane domain, or a linker. The hinge-region may have one or more rigid and/or flexible segments. A hinge-region may enhance the flexibility of the construct at the site of the hinge-region. The hinge-region may further help to reduce spatial constraints between the engineered protein binding domain and the rest of a fusion protein/construct. In one embodiment, the hinge-region is present in combination with a linker. In another embodiment, the hinge-region is present without a linker. In some embodiments, the hinge-region is a hinge-region derived from CD8 and/or CD28, such as a hinge-region comprising an amino acid sequence having a sequence identity of at least 70%, preferably at least 80%, more preferably at least 90%, and most preferably at least 95% sequence identity, to an amino acid sequence selected from any one of SEQ ID NOs: 46 to 49. In one embodiment, the linker is rich in glycine for flexibility. In one embodiment, the linker is rich serine or threonine for solubility.

The term "linker", as used herein, shall refer to a structural protein domain that has one or more rigid and/or flexible segments. According to this invention, when the immunomodulatory molecule is a CAR, the linker is located between the hinge-region and the transmembrane domain. According to this invention, when the immunomodulatory molecule is a bispecific molecule, the linker is located between the at least two binding domains. In some embodiments, the linker is present in combination with a hinge-region. In other embodiments, the linker is present without a hinge-region. In some embodiments, the linker comprises an amino acid sequence having a sequence identity of at least 70%, preferably at least 80%, more preferably at least 90%, and most preferably at least 95% sequence identity, to an amino acid sequence selected from any one of SEQ ID NOs: 41, 42 and 91. In some embodiments, ther term "linker" as used herein refers to both a hinge-region and an additonal linker. In other embodiments, the term "linker" as used herein refers to a linker in absence of a hinge-region.

The term "single-chain variable fragment (scFv)", or "antibody-derived single-chain variable fragment (scFv)", as used herein, shall refer to a fusion protein of the variable regions of the heavy chains (VH) and the light chains (VL) of immunoglobulins, connected with a short linker of about 10 to 25 amino acids in length. The linker is usually rich in glycine for flexibility, as well as serine or threonine for solubility, and can either connect the N-terminus of the VH with the C-terminus of the VL, or vice versa. According to this invention, the term "single-chain variable fragment" shall refer to a fragment that retains the specificity of the original immunoglobulin, despite removal of the constant regions and the introduction of the linker. In an scFv, an antigen-binding domain is typically expressed as a single peptide. scFvs can be created directly from subcloned heavy and light chains derived from a hybridoma.

The terms "identical" or percent "identity," in the context of two or more polypeptide sequences, refer to two or more sequences or subsequences that are the same or have a specified percentage of amino acid residues that are the same (i.e., 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection (see, e.g., NCBI web site http://www.ncbi.nlm.nih.gov/BLAST/ or the like). Such sequences are then said to be "substantially identical." The definition also includes sequences that have deletions and/or additions, as well as those that have substitutions. As described below, the preferred algorithms can account for gaps and the like. Preferably, identity exists over a region that is, for example, at least about 4,5, 6, 7, or 8, amino acids in length.

The terms "polypeptide," "peptide" and "protein" are used interchangeably herein to refer to a polymer of amino acid residues. The terms apply also to amino acid polymers in which one or more amino acid residue is an artificial chemical mimetic of a corresponding naturally occurring amino acid, as well as to naturally occurring amino acid polymers and non-naturally occurring amino acid polymers.

The term "amino acid" refers to naturally occurring and synthetic amino acids, as well as amino acid analogues and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acids may be referred to herein by either their commonly known three letter symbols or by the one-letter symbols recommended by the IUPAC-IUB Biochemical Nomenclature Commission.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" or "derivative", where the alteration results in the substitution of an amino acid, e.g., with a chemically similar amino acid. Conservative substitution tables providing functionally similar amino acids are well known in the art.

The term "isolated" refers to material that is substantially or essentially free or purified from components that normally accompany it in its native state. For example, the nucleic acid according to the invention may be modified subsequent to isolation from its natural or laboratory-produced environment, or it may be used in isolated form in vitro, or as components of devices, compositions, etc.

According to the present invention, the terms "obtaining", "producing", and "generating", can be used interchangeably. As such, a "method of obtaining a protein binding molecule", can also be referred to as a "method of producing a protein binding molecule", or a "method of generating a protein binding molecule". Similarly, a "method of obtaining a chimeric antigen receptor (CAR) expressing immune effector cell product, such as a CAR-T cell product, and/or a HLA-independent T cell receptor (HIT) expressing immune effector cell product, such as a HIT-T cell product, and/or a bispecific molecule expressing immune effector cell product, such as a bispecific T cell engager (BiTE) expressing immune effector cell product, comprising an immunomodulatory molecule, and/or a bispecific molecule, such as a bispecific T cell engager (BiTE), comprising an engineered protein binding domain", can also be referred to as a "method of producing a CAR expressing immune effector cell product, such as a CAR-T cell product, and/or a HLA-independent T cell receptor (HIT) expressing immune effector cell product, such as a HIT-T cell product, and/or a bispecific molecule expressing immune effector cell product, such as a BiTE expressing immune effector cell product, comprising an immunomodulatory molecule, and/or a bispecific molecule, such as a BiTE, comprising an engineered protein binding domain", or a "method of generating a CAR expressing immune effector cell product, such as a CAR-T cell product, and/or a HLA-independent T cell receptor (HIT) expressing immune effector cell product, such as a HIT-T cell product, and/or a bispecific molecule expressing immune effector cell product, such as a BiTE expressing immune effector cell product, comprising an immunomodulatory molecule, and/or a bispecific molecule, such as a BiTE, comprising an engineered protein binding domain".

According to the present invention, a HLA-independent T cell receptor (HIT) expressing immune effector cell product, such as a HIT-T cell product, is a HLA-independent T cell receptor (HIT) expressing immune effector cell product, such as a HIT-T cell product, as described in [4] (Mansilla-Soto, J. *et al.* 2022).

The term "patient" as used herein, refers to the recipient of a treatment. For example, a patient is the recipient of an immunomodulatory molecule according to the first aspect of this invention, an engineered protein binding domain according to the second aspect of this invention, an isolated nucleic acid according to the third aspect of this invention, a product according to the fifth aspect of this invention, an immunomodulatory molecule, isolated nucleic acid, or product according to the sixth aspect of the present invention, or a pharmaceutical composition according to the seventh aspect of this invention. In a specific embodiment, the patient is a mammal, such as a human, canine, murine, feline, bovine, ovine, swine or caprine. In a preferred embodiment, the patient is a human, such as a human suffering from a disease.

The term "pharmaceutically acceptable excipient(s) and/or carrier(s)" is meant to include a solid or liquid filler, stabilizer, diluent or encapsulating substance that can be safely used in administration routes when applied to an animal, e.g. a mammal, including humans.

The term "therapeutic treatment" and "treatment" refers to any type of therapy referred to herein, including the treatment to prevent an organ transplant rejection. Further included is the treatment of any kind of disease, such as a disease selected from cancer, autoimmune diseases, infectious diseases, cardiovascular diseases, cerebrovascular diseases, neurodegenerative diseases, dermatological diseases, genetic diseases, aging-related diseases, nephrological diseases, endocrinological diseases, fibrotic diseases, metabolic diseases, obesity-related diseases, gynecological diseases, ophthalmic diseases, otorhinolaryngologic diseases, allergies, and an immune deficiency.

Below, embodiments of the invention are disclosed. When the expression "and/or" is used, this means that each member of a respective list may be analyzed or used individually, or that more than one member of said list may be used. Further, when a list of members is combined with another list or lists of members, this means that each and every possible combination is encompassed by the present invention even if not every combination of the lists is explicitly, i.e. literally disclosed.

In the first aspect, the invention pertains to an immunomodulatory molecule, comprising an engineered protein binding domain, a linker and/or a hinge-region, and a domain for the activation and/or stimulation of an immune effector cell;
wherein the engineered protein binding domain comprises two or three alpha-helices and has a length of 59-100 amino acids, preferably of 60-90 amino acids, even more preferably of 60-80 amino acids, even more preferably of 60-70 amino acids, and most preferably of 60-65 amino acids;
wherein, optionally, the engineered protein binding domain contains one or more beta-sheet structures;
wherein the engineered protein binding domain is not derived from an animal or a human; wherein the engineered protein binding domain is not derived from a natural protein binding molecule, such as an antibody or a DARPin; wherein the engineered protein binding domain is not the result of a ribosome/phage display technique; and wherein the engineered protein binding domain is not an affibody.

The inventors surprisingly found that by employing computational and generative target-to-binder models for rapid and straightforward design of de novo protein binders, and incorporating these de novo protein binders into CARs, HITs, and/or BiTEs, immunomodulatory molecules can be designed and generated in an extremely fast and easy way, e.g. within minutes. The de novo binders, i.e. immunomodulatory molecules comprising an engineered protein binding domain according to the present invention, can be generated within minutes with computational methods, e.g., using artificial intelligence (AI), and can then be tested in a cell therapy context. Accordingly, the de novo binders, i.e. immunomodulatory molecules comprising an engineered protein binding domain according to the present invention, are generated in a fast and cheap way. It is a further advantage of the computationally designed binders (immunomodulatory molecules comprising an engineered protein binding domain according to the present invention) that they have a substantially smaller size compared to CARs, HITs, or BiTEs that use an scFv. This makes the immunomodulatory molecules comprising an engineered protein binding domain according to the present invention better suited for ex vivo manufacturing of immune therapies and in vivo delivery into immune cells (e.g., via mRNA).

Moreover, the immunomodulatory molecules comprising an engineered protein binding domain according to the present invention are less likely to induce patient immune responses than scFvs derived from antibodies raised in animals or bacterial cell systems (i.e. by phage display). Such antibodies raised in animals or bacterial cell systems are often subjected to a humanization procedure to reduce the immunogenicity of these antibodies, which is lengthy and costly. The immunomodulatory molecules comprising an engineered protein binding domain according to the present invention do not require such a humanization procedure, which further reduces the time and costs of the method of generating such immunomodulatory molecules. Moreover, the immunomodulatory molecules comprising an engineered protein binding domain according to the present invention have a reduced immunogenicity compared to conventionally used immunomodulatory molecules. Accordingly, due to the reduced immunogenicity of the immunomodulatory molecules comprising an engineered protein binding domain according to the present invention will help to make immune therapies derived therefrom persist longer in a patient administered immunomodulatory molecules according to the present invention. Thus, the therapeutic function of the administered immunomodulatory molecules will be employed for a longer period of time after administration.

It is a further advantage of the present invention that protein binding domains having a length of 59-100 amino acids, preferably of 60-90 amino acids, even more preferably of 60-80 amino acids, even more preferably of 60-70 amino acids, and most preferably of 60-65 amino acids, can be delivered and expressed in a very fast way. Thus, the de novo binders (immunomodulatory molecules comprising an engineered protein binding domain) according to the present invention are characterized by an enhanced delivery and expression.

In one embodiment, the engineered protein binding domain does not comprise an ankyrin repeat motif.

In a preferred embodiment, the domain for the activation and/or stimulation of an immune effector cell is an intracellular domain for the activation and/or stimulation of the immune effector cell. In another embodiment, the domain for the activation and/or stimulation of an immune effector cell is a domain binding to an immune effector cell and thereby activating and/or stimulating the immune effector cell. In a further preferred embodiment, the domain for the activation and/or stimulation of an immune effector cell is a domain specifically binding to an immune effector cell and thereby activating and/or stimulating the immune effector cell.

In another preferred embodiment, which can be combined with all other embodiments and aspects of the present invention, the immune effector cell is selected from a group comprising T cells, natural killer (NK) cells, natural killer T (NKT) cells, lymphocytes, myeloid cells, such as eosinophils, mast cells, basophils, granulocytes, macrophages, and any of the abovementioned cells derived from human induced pluripotent stem cells (hiPSCs); wherein, preferably, the immune effector cell is a T cell, an NK cell, or a macrophage; wherein, more preferably, the immune effector cell is a T cell.

In one embodiment, the engineered protein binding domain binds to an antigen selected from a group comprising tumor-associated antigens, tumor-specific antigens, antigens associated with a disease, antigens associated with toxin removal, and antigens associated with organ transplant rejection; wherein, preferably, the engineered protein binding domain binds to a tumor-associated antigen;
wherein, preferably, the tumor-associated antigens are selected from a group comprising: Epidermal growth factor receptor (EGFR), B-cell maturation antigen (BCMA, TNFRSF17), CD3, CD5, CD7, B-lymphocyte antigen CD19 (CD19), B-lymphocyte antigen CD20 (CD20), CD22, CD37, CAMPATH-1 antigen (CD52), CD70, CD79b, epidermal growth factor receptor variant III (EGFRvIII), Fibroblast activation protein (FAP), Fibroblast activation protein alpha (FAPalpha), G-protein coupled receptor family C group 5 member D (GPRC5D), Receptor tyrosine-protein kinase erbB-2 (HER2, ERBB2), Interleukin-13 receptor subunit alpha-2 (IL-13Ra2, CD213A2), Mesothelin (MSLN), Mucin short variant S1 (MUC1), Mucin-16 (MUC16, CA-125), prostate-specific membrane antigen (PSMA, GCPII), Prostate stem cell antigen (PSCA), Tyrosine-protein kinase transmembrane receptor ROR1 (ROR1, NTRKR1), SLAM family member 7 (SLAMF7, CD319), Transmembrane activator and CAML interactor (TACI, TNFRSF13B), and Vascular endothelial growth factor (VEGF); more preferably EGFR, BCMA, TACI, CD19, CD3, FAP, and HER2; even more preferably EGFR, BCMA, TACI, CD19, and CD3; and most preferably EGFR and BCMA;
wherein, preferably, the engineered protein binding domain binds to a cell surface antigen;
wherein, preferably, the tumor-specific antigen is epidermal growth factor receptor variant III (EGFRvIII); and
wherein, preferably, the antigens associated with a disease are antigens associated with an autoimmune disease, an infectious disease, a cardiovascular disease, a cerebrovascular disease, a neurodegenerative disease, a dermatological disease, a genetic disease, an aging-related disease, a nephrological disease, an endocrinological disease, a fibrotic disease, a metabolic disease, an obesity-related disease, a gynecological disease, an ophthalmic disease, an otorhinolaryngologic disease, an allergy, and/or an immune deficiency.

One embodiment relates to the immunomodulatory molecule according to the first aspect of this invention, wherein the immunomodulatory molecule is a chimeric antigen receptor (CAR), a HLA-independent T cell receptor (HIT), or a bispecific molecule, such as a bispecific T cell engager (BiTE); wherein, optionally the immunomodulatory molecule contains modifications in the CAR, the HIT, or the bispecific molecule to modulate the potency of an activation and/or stimulation of an immune effector cell, such as to reduce T cell exhaustion.

In one embodiment, the term "modulate the potency of an activation and/or stimulation of an immune effector cell" refers to reducing T cell exhaustion.

In one embodiment, a binding kinetic of a binding of the engineered protein binding domain to a target of the engineered protein binding domain is different from a binding kinetic of a binding of an antibody-derived single-chain variable fragment (scFv) binding domain to the same target; wherein, preferably, the binding kinetic of the engineered protein binding domain is slower or faster than the binding kinetic of the scFv binding domain; wherein, more preferably, the binding kinetic of the engineered protein binding domain is slower than the binding kinetic of the scFv binding domain.

In one embodiment, a binding avidity and/or affinity of a binding of the engineered protein binding domain to a target of the engineered protein binding domain is different from a binding avidity and/or affinity of a binding of an scFv binding domain to the same target; wherein, preferably, the binding avidity and/or affinity of the engineered protein binding domain is lower or higher than the binding avidity and/or affinity of the scFv binding domain; wherein, more preferably, the binding avidity and/or affinity of the engineered protein binding domain is lower than the binding avidity and/or affinity of the scFv binding domain.

In one embodiment, a cytotoxicity of the immunomodulatory molecule is lower than a cytotoxicity of an immunomodulatory molecule containing one or more scFv binding domains directed at the same target as the engineered protein binding domain of the immunomodulatory molecule.

In one embodiment, a cytokine production of the immunomodulatory molecule is comparable and/or higher than a cytokine production of an immunomodulatory molecule containing one or more scFv binding domains directed at the same target as the engineered protein binding domain of the immunomodulatory molecule.

Another embodiment relates to the immunomodulatory molecule according to the first aspect of this invention, wherein the immunomodulatory molecule is a CAR, and the CAR comprises:
(a) an engineered protein binding domain comprising two or three alpha-helices and 59-100 amino acids; preferably 60-90 amino acids; even more preferably 60-80 amino acids, even more preferably 60-70 amino acids; most preferably 60-65 amino acids; wherein, optionally, the engineered protein binding domain contains one or more beta-sheet structures or wherein the engineered protein binding domain contains no beta-sheet structures;
(b) a transmembrane domain;
(c) a hinge-region connecting the (a) engineered protein binding domain and the (b) transmembrane domain;
(d) optionally, a linker connecting the (a) engineered protein binding domain and the (c) hinge-region; and
(e) a domain for the activation and/or stimulation of an immune effector cell, wherein the domain for the activation and/or stimulation of an immune effector cell is an intracellular domain that activates and/or stimulates the immune effector cell upon binding of the engineered protein binding domain to a target region.

Another embodiment relates to the immunomodulatory molecule according to the first aspect of this invention, wherein the immunomodulatory molecule is a CAR, and wherein the hinge-region has a length of 10 to 200 amino acids, preferably of 20 to 100 amino acids;
wherein, optionally, the hinge-region comprises an amino acid sequence having a sequence identity of at least 70%, preferably at least 80%, more preferably at least 90%, and most preferably at least 95% sequence identity, to an amino acid sequence selected from SEQ ID NOs: 46 to 49, or, in each case independently, optionally with no more than ten, nine, eight, seven, six, five, four, preferably no more than three, two or one, amino acid substitution(s), insertion(s) or deletion(s) compared to these sequences.

In one embodiment relating to the immunomodulatory molecule according to the first aspect of this invention, the immunomodulatory molecule is a CAR, and the linker has a length of 0 to 100 amino acids; preferably 0 to 33 amino acids; wherein, optionally, the linker has one or more rigid and/or flexible segments;
wherein, optionally, the linker comprises an amino acid sequence having a sequence identity of at least 70%, preferably at least 80%, more preferably at least 90%, and most preferably at least 95% sequence identity, to an amino acid sequence selected from SEQ ID NOs: 41 and 42, or to an amino acid sequence encoded by the nucleotide sequence selected from SEQ ID NOs: 43 and 44, or, in each case independently, optionally with no more than ten, nine, eight, seven, six, five, four, preferably no more than three, two or one, amino acid substitution(s), insertion(s) or deletion(s) compared to these sequences.

In one embodiment relating to the immunomodulatory molecule according to the first aspect of this invention, the immunomodulatory molecule is a CAR, and the linker has a length of 0 to 100 amino acids; preferably 0 to 33 amino acids; wherein, optionally, the linker has one or more rigid and/or flexible segments;
wherein, optionally, the linker comprises an amino acid sequence having a sequence identity of at least 70%, preferably at least 80%, more preferably at least 90%, and most preferably at least 95% sequence identity, to an amino acid sequence of SEQ ID NO: 91, or to an amino acid sequence encoded by the nucleotide sequence of SEQ ID NO: 92, or, in each case independently, optionally with no more than ten, nine, eight, seven, six, five, four, preferably no more than three, two or one, amino acid substitution(s), insertion(s) or deletion(s) compared to these sequences. In this embodiment, the engineered protein binding domain may comprise or comprises at least two engineered protein binders, such as two, or three, or four, or five, or six, or seven, or eight, or nine, or ten, or any number of engineered protein binders, because the linker comprising an amino acid sequence having a sequence identity of at least 70%, preferably at least 80%, more preferably at least 90%, and most preferably at least 95% sequence identity, to an amino acid sequence of SEQ ID NO: 91, or to an amino acid sequence encoded by the nucleotide sequence of SEQ ID NO: 92, or, in each case independently, optionally with no more than ten, nine, eight, seven, six, five, four, preferably no more than three, two or one, amino acid substitution(s), insertion(s) or deletion(s) compared to these sequences, can combine the at least two engineered protein binders, such as the two, or three, or four, or five, or six, or seven, or eight, or nine, or ten, or any number of engineered protein binders. Of note, a linker comprising an amino acid sequence having a sequence identity of at least 70%, preferably at least 80%, more preferably at least 90%, and most preferably at least 95% sequence identity, to an amino acid sequence of SEQ ID NO: 91 (which can be encoded by a nucleotide sequence according to SEQ ID NO: 92), is sometimes referred to as Whitlow linker.

In one embodiment, the engineered protein binding domain comprises at least two engineered protein binders, such as two, or three, or four, or five, or six, or seven, or eight, or nine, or ten, or any number of engineered protein binders, such as two, or three, or four, or five, or six, or seven, or eight, or nine, or ten, or any number of engineered protein binding domains that bind to EGFR and/or engineered protein binding domains that bind to BCMA, optionally wherein the engineered protein binding domain that binds to EGFR comprises an amino acid sequence having a sequence identity of at least 70%, more preferably at least 80%, even more preferably at least 90%, and most preferably at least 95% sequence identity, to an amino acid sequence selected from any one of SEQ ID NOs: 1 to 22, or to an amino acid sequence encoded by the nucleotide sequence selected from any one of SEQ ID NOs: 50 to 71, or, in each case independently, optionally with no more than ten, nine, eight, seven, six, five, four, preferably no more than three, two or one, amino acid substitution(s), insertion(s) or deletion(s) compared to these sequences; or wherein the engineered protein binding domain that binds to BCMA comprises an amino acid sequence having a sequence identity of at least 70%, more preferably at least 80%, even more preferably at least 90%, and most preferably at least 95% sequence identity, to an amino acid sequence selected from any one of SEQ ID NOs: 23 to 40, or to an amino acid sequence encoded by the nucleotide sequence selected from any one of SEQ ID NOs: 72 to 89, or, in each case independently, optionally with no more than ten, nine, eight, seven, six, five, four, preferably no more than three, two or one, amino acid substitution(s), insertion(s) or deletion(s) compared to these sequences.

In one embodiment, the engineered protein binding domain comprises at least two engineered protein binders, such as two, or three, or four, or five, or six, or seven, or eight, or nine, or ten, or any number of engineered protein binders, such as two, or three, or four, or five, or six, or seven, or eight, or nine, or ten, or any number of engineered protein binding domains that bind to EGFR and/or engineered protein binding domains that bind to BCMA, and the linker comprises an amino acid sequence having a sequence identity of at least 70%, preferably at least 80%, more preferably at least 90%, and most preferably at least 95% sequence identity, to an amino acid sequence selected from SEQ ID NOs: 41, 42, and 91, or to an amino acid sequence encoded by the nucleotide sequence selected from SEQ ID NOs: 43, 44, and 92 or, in each case independently, optionally with no more than ten, nine, eight, seven, six, five, four, preferably no more than three, two or one, amino acid substitution(s), insertion(s) or deletion(s) compared to these sequences.

In one embodiment, the engineered protein binding domain comprises at least two engineered protein binders, such as two, or three, or four, or five, or six, or seven, or eight, or nine, or ten, or any number of engineered protein binders, such as two, or three, or four, or five, or six, or seven, or eight, or nine, or ten, or any number of engineered protein binding domains that bind to EGFR and/or engineered protein binding domains that bind to BCMA, and the linker comprises an amino acid sequence having a sequence identity of at least 70%, preferably at least 80%, more preferably at least 90%, and most preferably at least 95% sequence identity, to the amino acid sequence of SEQ ID NO: 91, or to an amino acid sequence encoded by the nucleotide sequence of SEQ ID NO: 92 or, in each case independently, optionally with no more than ten, nine, eight, seven, six, five, four, preferably no more than three, two or one, amino acid substitution(s), insertion(s) or deletion(s) compared to these sequences.

Importantly, CAR-T properties and functions can be modulated using linkers having different properties, such as rigid vs. flexible linkers. Thus, CAR-T properties and functions can be specifically tailored by using linkers having one or more rigid and/or flexible segments.

In one embodiment relating to the immunomodulatory molecule according to the first aspect of this invention, which can be combined with every embodiment and/or aspect of this invention, the linker is rich in glycine for flexibility. In one embodiment relating to the immunomodulatory molecule according to the first aspect of this invention, which can be combined with every embodiment and/or aspect of this invention, the linker is rich serine or threonine for solubility.

In one embodiment relating to the immunomodulatory molecule according to the first aspect of this invention, the immunomodulatory molecule is a CAR, and wherein the transmembrane domain (TM) is selected from a group comprising the TMs of CD8, CD28, an alpha, beta or zeta chain of a T cell receptor, CD3 epsilon, CD45, CD4, CD5, CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRFI), CD160, CD19, IL2R beta, IL2R gamma, IL7R, ITGA1 (CD49a, VLA1), ITGA4 (IA4, CD49D), ITGA6(VLA-6)F, CD49, ITGAD, CD1 1d, ITGAE, CD103, ITGAL, CDI la, LFA-1, ITGAM, CDI Ib, ITGAX, CDI Ic, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), SLAMF6 (NTB-A, Lylo8), SLAM (SLAMF1, CD150, IPO-3), BLAME (SLAMF8), SELPLG (CD162), LTBR, PAG/Cbp, NKp44, NKp30, NKp46, NKG2D, and/or NKG2C; preferably the TM of CD8 or CD28; more preferably the TM of CD8.

In one embodiment, the immunomodulatory molecule is a CAR, and the intracellular domain that activates and/or stimulates the immune effector cell upon binding of the engineered protein binding domain to a target region comprises a signaling domain selected from a group comprising the signaling domains of CD3 zeta, TCR zeta, FcR gamma, FcR beta, CD3 gamma, CD3 theta, CD3 delta, CD3 epsilon, CD22, CD79a, CD79b, and CD66d; preferably the signaling domains of CD3 zeta; and
wherein, optionally, the intracellular domain that activates and/or stimulates the immune effector cell upon binding of the engineered protein binding domain to a target region further comprises one or more co-stimulatory domains; wherein, preferably, the one or more co-stimulatory domains is/are selected from a group comprising the co-stimulatory domains of 4-1BB (CD137), CD28, CARD11, CD2, CD7, CD27, CD30, CD40, CD54 (ICAM), CD83, CD134 (OX40), CD150 (SLAMF1), CD152 (CTLA4), CD223 (LAG3), CD270 (HVEM), CD273 (PD-L2), CD274 (PD-L1), CD278 (ICOS), DAP10, LAT, NKD2C SLP76, TRIM, and ZAP70; preferably selected from the co-stimulatory domains of 4-1BB (CD137) and CD28; wherein, even more preferably the co-stimulatory domain is the co-stimulatory domain of 4-1BB (CD137).

One embodiment relates to the immunomodulatory molecule according to the first aspect of this invention, wherein the immunomodulatory molecule is a HIT receptor.

In one embodiment, a HIT receptor is generated in situ by knocking an engineered protein binding domain into the TCR-alpha constant (TRAC) and/or the (TRBC) gene locus.

In one embodiment, a HIT receptor is generated in situ by knocking an engineered protein binding domain into the TRAC and/or TRBC gene locus, thereby generating an engineered protein binding domain that is fused to the endogenous T cell receptor constant beta (TCR Cβ) domain, and/or is fused to the endogenous T cell receptor constant alpha (TRC Ca) domain.

One embodiment relates to the immunomodulatory molecule according to the first aspect of this invention, wherein the immunomodulatory molecule is a HIT receptor, and the HIT receptor comprises:
(a) an engineered protein binding domain comprising two or three alpha-helices and 59-100 amino acids; preferably 60-90 amino acids; even more preferably 60-80 amino acids, even more preferably 60-70 amino acids; most preferably 60-65 amino acids; wherein, optionally, the engineered protein binding domain contains one or more beta-sheet structures or wherein the engineered protein binding domain contains no beta-sheet structures;
(b) an engineered protein binding domain that is fused to the endogenous T cell receptor constant beta (TCR Cβ) domain, and/or is fused to the endogenous T cell receptor constant alpha (TRC Ca) domain, and
(c) optionally, a linker sequence connecting an engineered protein binding domain to the endogenous TCR Cβ and/or TRC Cα domain can be employed.

Another embodiment relates to the immunomodulatory molecule according to the first aspect of this invention, wherein the immunomodulatory molecule is a HIT, and wherein the hinge-region has a length of 10 to 200 amino acids, preferably of 20 to 100 amino acids;
wherein, optionally, the hinge-region comprises an amino acid sequence having a sequence identity of at least 70%, preferably at least 80%, more preferably at least 90%, and most preferably at least 95% sequence identity, to an amino acid sequence selected from SEQ ID NOs: 46 to 49, or, in each case independently, optionally with no more than ten, nine, eight, seven, six, five, four, preferably no more than three, two or one, amino acid substitution(s), insertion(s) or deletion(s) compared to these sequences.

Importantly, properties and functions of HITs can be modulated using linkers having different properties, such as rigid vs. flexible linkers. Thus, properties and functions of HITs can be specifically tailored by using linkers having one or more rigid and/or flexible segments.

In one embodiment relating to the immunomodulatory molecule according to the first aspect of this invention, the immunomodulatory molecule is a HIT, and the linker has a length of 0 to 100 amino acids; preferably 0 to 33 amino acids; wherein, optionally, the linker has one or more rigid and/or flexible segments;
wherein, optionally, the linker comprises an amino acid sequence having a sequence identity of at least 70%, preferably at least 80%, more preferably at least 90%, and most preferably at least 95% sequence identity, to an amino acid sequence selected from SEQ ID NOs: 41, 42, and 91, or to an amino acid sequence encoded by the nucleotide sequence selected from SEQ ID NOs: 43, 44, and 92 or, in each case independently, optionally with no more than ten, nine, eight, seven, six, five, four, preferably no more than three, two or one, amino acid substitution(s), insertion(s) or deletion(s) compared to these sequences.

In one embodiment relating to the first aspect of this invention, the immunomodulatory molecule is a bispecific molecule, such as a bispecific T cell engager (BiTE), and wherein the bispecific molecule comprises at least two binding domains,
wherein the first binding domain is an engineered protein binding domain comprising two or three alpha-helices and 59-100 amino acids; preferably 60-90 amino acids; even more preferably 60-80 amino acids, even more preferably 60-70 amino acids; most preferably 60-65 amino acids; wherein, optionally, the engineered protein binding domain contains one or more beta-sheet structures or wherein the engineered protein binding domain contains no beta-sheet structures; and
wherein the second binding domain is a domain for the activation and/or stimulation of an immune effector cell by specifically binding to an immune effector cell and thereby activating and/or stimulating the immune effector cell; and
wherein the linker and/or the hinge-region has a length of 5 to 200 amino acids; wherein optionally, the linker and/or the hinge-region has one or more rigid and/or flexible segments.

Importantly, properties and functions of bispecific molecules, such as bispecific T cell engagers (BiTEs) can be modulated using linkers having different properties, such as rigid vs. flexible linkers. Thus, properties and functions of bispecific molecules, such as bispecific T cell engagers (BiTEs), can be specifically tailored by using linkers having one or more rigid and/or flexible segments.

In one embodiment, the immunomodulatory molecule comprises an engineered protein binding domain targeting and/or binding to EGFR and/or BCMA, optionally wherein the engineered protein binding domain comprises an amino acid sequence according to SEQ ID NO: 1 or 2.

In one embodiment, the immunomodulatory molecule comprises an engineered protein binding domain targeting and/or binding to EGFR and/or BCMA, optionally wherein the engineered protein binding domain comprises an amino acid sequence according to any one of SEQ ID NOs: 3 to 40.

In one embodiment, the immunomodulatory molecule does not comprise an amino acid sequence according to SEQ ID NO: 1 or 2, and/or does not comprise an engineered protein binding domain having an amino acid sequence according to SEQ ID NO: 1 or 2.

In one embodiment relating to an immunomodulatory molecule according to the first aspect of this invention, the engineered protein binding domain is lighter, smaller, and/or shorter than an antibody-derived single-chain variable fragment (scFv) binding domain.

A second aspect of the present invention, which can be combined with any aspect and/or embodiment of the present invention, relates to an engineered protein binding domain, wherein said engineered protein binding domain is removed from an immunomodulatory molecule according to the first aspect of this invention, and wherein said engineered protein binding domain is used outside of the immunomodulatory molecule, optionally wherein said engineered protein binding domain is fused to an Fc-part or a CH3 domain of an antibody, and/or wherein said engineered protein binding domain is used as a binder drug conjugate.

A third aspect of the present invention, which can be combined with any aspect and/or embodiment of the present invention, relates to an isolated nucleic acid comprising a sequence encoding for an immunomodulatory molecule according to the fist aspect of this invention, or encoding for an immunomodulatory molecule according to the second aspect of this invention, or an expression construct for expressing the immunomodulatory molecule according to the first aspect of this invention in a (host) cell, preferably further comprising a promoter and/or a terminator sequence.

In a preferred embodiment, an expression vector containing a nucleotide sequence of a CAR can be transfected in an immune effector cell, such as a T cell, resulting in a CAR expressing immune effector cell product, such as a CAR-T cell product.

A fourth aspect of the present invention, which can be combined with any aspect and/or embodiment of the present invention, pertains to a method of obtaining a chimeric antigen receptor (CAR) expressing immune effector cell product, such as a CAR-T cell product, and/or a HLA-independent T cell receptor (HIT) expressing immune effector cell product, such as a HIT-T cell product, and/or a bispecific molecule expressing immune effector cell product, such as a bispecific T cell engager (BiTE) expressing immune effector cell product, comprising an immunomodulatory molecule, and/or a bispecific molecule, such as a bispecific T cell engager (BiTE), comprising an engineered protein binding domain; wherein optionally the immunomodulatory molecule is the immunomodulatory molecule according to the first aspect of this invention, and/or the engineered protein binding domain is as defined in any of the embodiments according to the first aspect of this invention; wherein the method comprises the steps:
i) identifying a target region in a target protein;
ii) running a computer program comprising instructions for implementing a method of identifying an engineered protein binding domain to the target region in the target protein, wherein the identification is a structure-based, machine learning, and/or diffusion-based identification method, wherein the step of running the computer program (ii) is repeated for a number of times, such as at least 10 times, at least 20 times, at least 30 times, at least 40 times, at least 50 times, at least 60 times, at least 70 times, at least 80 times, at least 90 times, or any number of times, preferably wherein the step of running the program is repeated at least 50 times, thereby obtaining at least one engineered protein binding domain candidate;
iii) pre-screening of the at least one engineered protein binding domain candidate, wherein the pre-screening comprises analyzing an alignment of the at least one engineered protein binding domain candidate to the target protein, and/or the binding of the at least one engineered protein binding domain candidate to the target protein;
iv) optionally, calculating the binding probability of the engineered protein binding domain candidate to the target protein;
v) selecting at least one engineered protein binding domain, wherein an engineered protein binding domain candidate with good alignment or binding to the target protein is classified as an engineered protein binding domain;
vi) generating at least one expression vector containing a nucleotide sequence of a CAR with one of the engineered protein binding domains; wherein, preferably, each expression vector contains a different nucleotide sequence of a CAR molecule and/or the engineered protein binding domain; and/or
vii) generating at least one expression vector containing a nucleotide sequence of a bispecific molecule with one of the engineered protein binding domains; wherein, preferably, each expression vector contains a different nucleotide sequence of a bispecific molecule and/or the engineered protein binding domain;
viii) transfecting one expression vector of step vi) and/or step vii) in an immune effector cell, such as a T cell, resulting in a CAR expressing immune effector cell product, comprising the engineered protein binding domain; or transfecting one expression vector of step vii) in a cell, such as a HEK293 cell, a HEK293T cell, a CHO cell, a HT-1080 cell, or a PER.C6 cell, resulting in a cell producing the bispecific molecule comprising the engineered protein binding domain; and
ix) optionally, isolating said bispecific molecule comprising the engineered protein binding domain;
thereby obtaining the CAR expressing immune effector cell product, such as the CAR-T cell product, and/or the HIT expressing immune effector cell product, such as the HIT-T cell product, and/or the bispecific molecule expressing immune effector cell product, such as the BiTE expressing immune effector cell product, comprising the immunomodulatory molecule, and/or the bispecific molecule, such as the BiTE.

The inventors surprisingly found a method for computationally designing binders for immune cell therapies, wherein de novo binders (immunomodulatory molecules comprising an engineered protein binding domain) are designed and generated in an extremely fast and easy way by employing computational and generative target-to-binder models for rapid and straightforward design of de novo protein binders, using the target protein sequence and structure as the primary input, and incorporating these de novo protein binders into CARs, HITs, and/or BiTEs.

In a preferred embodiment relating to the method of the fourth aspect of this invention, which can be combined with any aspect and/or embodiment of the present invention, the engineered protein binding domain is an engineered protein binding domain that binds to the target protein. Preferably, the engineered protein binding domain is an engineered protein binding domain that binds to the target region in the target protein.

In one embodiment, the step i) of identifying a target region in a target protein is a computational design that is based on the structure of the target protein and/or is based on a rational choice of potential hotspot residues within the target protein.

In one embodiment relating to the fourth aspect of this invention, the target region is a flat, plain, and/or hydrophobic target region. In one embodiment relating to the fourth aspect of this invention, the target region comprises at least one beta sheet. In one embodiment relating to the fourth aspect of this invention, the target region is an accessible target region and/or is not buried in a cell membrane. In one embodiment, the term "accessible" refers to a target region that is not buried in a cell membrane and/or is located on the outside of a cell. In one embodiment relating to the fourth aspect of this invention, the target region does not comprise a salt bridge.

In one embodiment, the step ii) of running a computer program comprising instructions for implementing a method of identifying an engineered protein binding domain to the target region in the target protein, is a diffusion-based identification method, wherein the diffusion-based identification method is implemented by RoseTTAFoldDiffusion (RF diffusion), which generates at least one engineered protein binding domain candidate.

In one embodiment, the step ii) of running a computer program comprising instructions for implementing a method of identifying an engineered protein binding domain to the target region in the target protein, is a diffusion-based identification method implemented by RoseTTAFoldDiffusion (RF diffusion), which generates at least one engineered protein binding domain candidate in a first step. In a second step, the sequence of the at least one engineered protein binding domain candidate is designed with Protein MPNN, and said sequence is validated with Alphafold2 in a third step.

RF diffusion is a method for discovering new bioactive compounds using machine learning algorithms. In RF Diffusion, the structural information of known bioactive compounds is used as a dataset, and new compounds are generated from this structural information through machine learning algorithms. These generated compounds are ranked based on their predicted bioactivity, and the most promising compounds can be selected. In simple terms, RF Diffusion is a method that uses machine learning algorithms to create new compounds from known bioactive compounds, providing efficient and accurate results.

ProteinMPNN is a deep learning-based protein sequence design method that can design new proteins with high accuracy. ProteinMPNN is trained on a protein databank comprising thousands of high-resolution structures.

Alphafold2 is an artificial intelligence algorithm that predicts the three-dimensional structure of proteins. Alphafold2 can predict the three-dimensional structure of a protein from its amino acid sequence with high accuracy. Importantly, Alphafold2 shows a high correlation when compared with experimentally determined structures, which is extremely important in the medical and pharmaceutical fields.

In one embodiment, the protein sequence designed by, e.g. ProteinMPNN, is not a repetitive, such as a highly repetitive, amino acid sequence.

In one embodiment, the pre-screening of the at least one engineered protein binding domain candidate in step iii) of the method refers to a virtual pre-screening.

In one embodiment, in step v) of the method, i.e. the step of selecting at least one engineered protein binding domain, an engineered protein binding domain candidate with good alignment or binding to the target protein is classified as an engineered protein binding domain if it has an RMSD-value of lower than 20, of lower than 15, of lower than 10, of lower than 5, or of around 1. In a preferred embodiment, an engineered protein binding domain candidate with good alignment or binding to the target protein is classified as an engineered protein binding domain in step v) of the method if it has an RMSD-value of lower than 10.

In one embodiment relating to the fourth aspect of this invention, the method further comprises the step of:
x) functionally testing the CAR expressing immune effector cell product, such as the CAR-T cell product, the HIT expressing immune effector cell product, such as the HIT-T cell product, the bispecific molecule expressing immune effector cell product, such as the BiTE expressing immune effector cell product, comprising the immunomodulatory molecule, and/or the bispecific molecule, such as the BiTE, comprising the engineered protein binding domain.

In one embodiment, the functionally testing in step x) of the method comprises measuring and/or analyzing a T cell signaling molecule, such as a luminescence-based readout of a nuclear factor of activated T cells (NFAT) activation and/or a cytokine or lytic protein produced by a T cell and/or the proliferation of a T cell in response to the targeted antigen and/or the death of cancer cells.

In one embodiment, the functionally testing in step x) of the method comprises a screening in cells, such as a JURKAT NFAT reporter cell line. As an exemplary embodiment, lenti virus production is performed in a 24-well plate, followed by transduction of lenti viruses in JURKAT NFAT reporter cell lines. After a brief expansion for 3 to 5 days, the transduction efficiency is normalized for the next step based on a fluorescent signal, such as an mCherry signal. Normalized Jurkats with target cells are incubated over night and a luminescence-based readout of NFAT activation is analyzed with the help of a plate reader. Subsequently, a validation in primary T-cells occurs (T-cell signaling readout post-binding, e.g., production of a cytokine/lytic protein and/or T cell proliferation and/or T cell cytotoxicity against cancer cells).

In one embodiment, the method according to the fourth aspect of this invention does not require an animal and/or a bacterial cell system.

A fifth aspect of the present invention, which can be combined with any aspect and/or embodiment of the present invention, pertains to a product obtainable by a method according to the fourth aspect of this invention.

A sixth aspect of the present invention, which can be combined with any aspect and/or embodiment of the present invention, pertains to an immunomodulatory molecule according to the first aspect of this invention, an engineered protein binding domain according to the second aspect of this invention, an isolated nucleic acid according to the third aspect of this invention, or a product according to the fifth aspect of the present invention, wherein the immunomodulatory molecule is a CAR; and either
A) wherein the immunomodulatory molecule comprises an engineered protein binding domain that binds to EGFR, a TM of CD8, a hinge-region of CD8, a linker having a length of 0 to 100 amino acids, preferably 0 to 33 amino acids, more preferably a linker having an amino acid sequence of any one of SEQ ID NOs: 41,42, or 91; and a co-stimulatory domain of 4-1BB (CD137); wherein, preferably, the engineered protein binding domain comprises an amino acid sequence having a sequence identity of at least 70%, more preferably at least 80%, even more preferably at least 90%, and most preferably at least 95% sequence identity, to an amino acid sequence selected from any one of SEQ ID NOs: 1 to 22, or to an amino acid sequence encoded by the nucleotide sequence selected from any one of SEQ ID NOs: 50 to 71, or, in each case independently, optionally with no more than ten, nine, eight, seven, six, five, four, preferably no more than three, two or one, amino acid substitution(s), insertion(s) or deletion(s) compared to these sequences; or
B) wherein the immunomodulatory molecule comprises an engineered protein binding domain that binds to BCMA, a TM of CD8, a hinge-region of CD8, a linker having a length of 0 to 100 amino acids, preferably 0 to 33 amino acids, more preferably a linker having an amino acid sequence of any one of SEQ ID NOs: 41,42, or 92; and a co-stimulatory domain of 4-1BB (CD137); wherein, preferably, the engineered protein binding domain comprises an amino acid sequence having a sequence identity of at least 70%, more preferably at least 80%, even more preferably at least 90%, and most preferably at least 95% sequence identity, to an amino acid sequence selected from any one of SEQ ID NOs: 23 to 40, or to an amino acid sequence encoded by the nucleotide sequence selected from any one of SEQ ID NOs: 72 to 89, or, in each case independently, optionally with no more than ten, nine, eight, seven, six, five, four, preferably no more than three, two or one, amino acid substitution(s), insertion(s) or deletion(s) compared to these sequences.

In one embodiment, an example CAR construct is encoded by the nucleotide sequence according to SEQ ID NO: 90: 
ATGGCCCTGCCCGTGACCGCCCTGCTGCTGCCCCTGGCCCTGCTGCTGCACGCCGCCAGGCCC
GATCACTGGGAGGAAGTGTTCAGATGGGCCCTGGAGCACCTGCAAGAGGCGACCCAGCAGAA
CGACCCTCAGAAAGCCAAGAAGATCCTGGAAGAAGCTCACAAGTGGCTCCGCCGCGAGCTGAG
CGAGGAAGAGGCCCGGGCCGTCGTGCGGTGGCTGAAGCAGCTGGTGGACAGAGAGCTGTCCA
CCACTACCCCAGCACCGAGGCCACCCACCCCGGCTCCTACCATCGCCTCCCAGCCTCTGTCCCT
GCGTCCGGAGGCATGTAGACCCGCAGCTGGTGGGGCCGTGCATACCCGGGGTCTTGACTTCGC
CTGCGATATCTACATTTGGGCCCCTCTGGCTGGTACTTGCGGGGTCCTGCTGCTTTCACTCGTG
ATCACTCTTTACTGTAAGCGCGGTCGGAAGAAGCTGCTGTACATCTTTAAGCAACCCTTCATGA
GGCCTGTGCAGACTACTCAAGAGGAGGACGGCTGTTCATGCCGGTTCCCAGAGGAGGAGGAA
GGCGGCTGCGAACTGCGCGTGAAATTCAGCCGCAGCGCAGATGCTCCAGCCTACCAACAGGGG
CAGAACCAGCTCTACAACGAACTCAATCTTGGTCGGAGAGAGGAGTACGACGTGCTGGACAAG
CGGAGAGGACGGGACCCAGAAATGGGCGGGAAGCCGCGCAGAAAGAATCCCCAAGAGGGCCT
 
GTACAACGAGCTCCAAAAGGATAAGATGGCAGAAGCCTATAGCGAGATTGGTATGAAAGGGG
AACGCAGAAGAGGCAAAGGCCACGACGGACTGTACCAGGGACTCAGCACCGCCACCAAGGAC
ACCTATGACGCTCTTCACATGCAGGCCCTGCCGCCTCGG
 In this embodiment, the CD8 leader sequence is underlined once with a straight line (nucleotides 1 to 63 of SEQ ID NO: 90), the engineered protein binder EGFR_n is underlined with a curly line (nucleotides 64 to 249 of SEQ ID NO: 90), the CD8 hinge-region and transmembrane domain sequence is not underlined (nucleotides 250 to 456 of SEQ ID NO: 90), the 4-1BB ICD sequence is underlined twice with straight lines (nucleotides 457 to 582 of SEQ ID NO: 90), and the CD3 zeta sequence is underlined with a dashed line (nucleotides 583 to 918 of SEQ ID NO: 90). In this embodiment, the example CAR construct is herein sometimes also referred to as "pTUMo011", or "pTUMo011 - CD8Leader/EGFRn_MiniProtBinder/CD8hingeTM/41BB/CD3z".

A seventh aspect of the present invention, which can be combined with any aspect and/or embodiment of the present invention, pertains to a pharmaceutical composition comprising the immunomodulatory molecule according to the first aspect of this invention, an engineered protein binding domain according to the second aspect of this invention, the isolated nucleic acid according to the third aspect of this invention, the product according to the fifth aspect of the present invention, or the immunomodulatory molecule, the isolated nucleic acid, or the product according to the sixth aspect of the present invention, wherein, optionally, said pharmaceutical composition comprises one or more pharmaceutically acceptable excipient(s) and/or carrier(s); wherein, further optionally, said pharmaceutical composition comprises one or more further agent(s) or drug(s), such as chemotherapeutic drugs, radioisotopes, antibody-drug conjugates, immune checkpoint inhibitors, bispecific molecules, such as BiTEs, BiTE-secreting cells, HITs, and/or CAR-T cells.

In one embodiment, an engineered protein binding domain of an immunomodulatory molecule according to the first aspect of this invention is removed from the immunomodulatory molecule and is used outside of the immunomodulatory molecule, optionally wherein said engineered protein binding domain is fused to an Fc-part or a CH3 domain of an antibody, and/or wherein said engineered protein binding domain is used as a binder drug conjugate.

In one embodiment, a binder drug conjugate is an antibody drug conjugate (ADC).

In one embodiment, an engineered protein binding domain of an immunomodulatory molecule according to the first aspect of this invention is removed from the immunomodulatory molecule and is used outside of the immunomodulatory molecule as or in a pharmaceutical composition, optionally wherein said engineered protein binding domain is fused to an Fc-part or a CH3 domain of an antibody, and/or wherein said engineered protein binding domain is used as a binder drug conjugate, optionally, wherein said pharmaceutical composition, said engineered protein binding domain fused to an Fc-part or a CH3 domain of an antibody, or said binder drug conjugate comprises one or more pharmaceutically acceptable excipient(s) and/or carrier(s); wherein, further optionally, said pharmaceutical composition, said engineered protein binding domain fused to an Fc-part or a CH3 domain of an antibody, or said binder drug conjugate comprises one or more further agent(s) or drug(s), such as chemotherapeutic drugs, radioisotopes, antibody-drug conjugates, immune checkpoint inhibitors, bispecific molecules, such as BiTEs, BiTE-secreting cells, HITs, and/or CAR-T cells.

In one embodiment, an immune checkpoint protein is preferentially selected from but not restricted to any one of PD-1, cd47, cd31, PD-L1, PD-L2, BTLA, HVEM, lag3, tigit, prv, cd155, cd112, galectin-9, tim3, tim4, gitr, gitrl, tigit, Vista, cd276, cd39, cd73, CTLA4, IL10, IL35, ido, vip, IL27, and IL37.

In a further embodiment, an immune checkpoint inhibitor is an antagonistic antibody or an antagonistic antibody fragment against an immune checkpoint protein or encoding an immune checkpoint inhibitor, such as an antibody selected from Atezolizumab, Avelumab, Cemiplimab, Dostarlimab, Durvalumab, Nivolumab, Pembrolizumab, Ipilimumab, and Relatlimab.

Atezolizumab (Tecentriq^{®}) is a checkpoint inhibitor that targets the PD-1/PD-L1 pathway, and is approved for subsets of patients with bladder cancer, breast cancer, liver cancer, lung cancer, and melanoma.

Avelumab (Bavencio^{®}) is a checkpoint inhibitor that targets the PD-1/PD-L1 pathway, and is approved for subsets of patients with bladder cancer, kidney cancer, and Merkel cell carcinoma, a type of skin cancer.

Cemiplimab (Libtayo^{®}) is a checkpoint inhibitor that targets the PD-1/PD-L1 pathway, and is approved for subsets of patients with cutaneous squamous cell carcinoma, basal cell carcinoma, and lung cancer.

Dostarlimab (Jemperli) is a checkpoint inhibitor that targets the PD-1 pathway, and is approved for subsets of patients with uterine (endometrial) cancer.

Durvalumab (Imfinzi^{™}) is a checkpoint inhibitor that targets the PD-1/PD-L1 pathway, and is approved for subsets of patients with bladder cancer and lung cancer.

Nivolumab (Opdivo^{®}) is a checkpoint inhibitor that targets the PD-1/PD-L1 pathway, and is approved for subsets of patients with bladder cancer, colorectal cancer, esophageal cancer, gastric cancer, head and neck cancer, kidney cancer, liver cancer, lung cancer, lymphoma, melanoma, and mesothelioma.

Pembrolizumab (Keytruda^{®}) is a checkpoint inhibitor that targets the PD-1/PD-L1 pathway, and is approved for subsets of patients with bladder cancer, breast cancer, cervical cancer, colorectal cancer, cutaneous squamous cell carcinoma, esophageal cancer, head and neck cancer, kidney cancer, liver cancer, lung cancer, lymphoma, melanoma, Merkel cell carcinoma, and stomach cancer. It is also approved to treat subsets of patients with cancers of any type that present with certain genetic mutations (MSI-H, dMMR, or TMB-H).

Ipilimumab (Yervoy^{®}) is a checkpoint inhibitor that targets the CTLA-4 pathway, and is approved for subsets of patients with melanoma, mesothelioma, liver cancer, and lung cancer.

Relatlimab is a checkpoint inhibitor that targets the LAG-3 pathway, and is approved in combination with nivolumab (together known as Opdualag^{™}) for subsets of patients with melanoma.

An eighth aspect of the present invention, which can be combined with any aspect and/or embodiment of the present invention, pertains to the immunomodulatory molecule according to the first aspect of this invention, the engineered protein binding domain according to the second aspect of this invention, the isolated nucleic acid according to the third aspect of this invention, the product according to the fifth aspect of this invention, the immunomodulatory molecule, the isolated nucleic acid, or the product according to the sixth aspect of the present invention, or the pharmaceutical composition according to the seventh aspect of this invention, for use in a method of diagnosis, prevention and/or treatment of a disease, or for preventing an organ transplant rejection, in a patient in need thereof; wherein, preferably, said patient is a mammal; wherein, more preferably, said patient is a human. The present invention is particularly advantageous for personalized therapies, e.g., in the context of cancer neoantigens.

One embodiment pertains to the immunomodulatory molecule, the engineered protein binding domain, the isolated nucleic acid, the product, or the pharmaceutical composition for use according to the eighth aspect of this invention, wherein the disease is selected from cancer, autoimmune diseases, infectious diseases, cardiovascular diseases, cerebrovascular diseases, neurodegenerative diseases, dermatological diseases, genetic diseases, aging-related diseases, nephrological diseases, endocrinological diseases, fibrotic diseases, metabolic diseases, obesity-related diseases, gynecological diseases, ophthalmic diseases, otorhinolaryngologic diseases, allergies, and an immune deficiency.

In another embodiment relating to the immunomodulatory molecule, the engineered protein binding domain, the isolated nucleic acid, the product, or the pharmaceutical composition for use according to the eighth aspect of this invention, the disease is cancer, and wherein the cancer is selected from one or more of brain tumor, esophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancers, skin cancer, leukemia, lymphoma, myeloma, plasma cell leukemia, plasma cell dyscrasia, myeloid malignancies, lymphoid malignancies, breast cancer, ovarian cancer, neuroendocrine carcinoma, endometrial cancer, vaginal cancers, blood-related cancers, uterine cancer, testicular cancer, glioma, bone sarcoma, cervix cancer, synovial sarcoma, and neuroendocrine carcinoma, for example high-grade poorly differentiated neuroendocrine carcinoma (NEC-G3); wherein, preferably, the cancer is selected from lung cancer, pancreatic cancer, colon cancer, rectal cancer, ovarian cancer, myeloma, plasma cell leukemia, and plasma cell dyscrasia; more preferably lung cancer, pancreatic cancer, colon cancer, rectal cancer, myeloma, and plasma cell leukemia; most preferably lung cancer, and myeloma; optionally wherein the cancer is a metastatic, stage III cancer, or stage IV cancer.

One embodiment pertains to the immunomodulatory molecule, the engineered protein binding domain, the isolated nucleic acid, the product, or the pharmaceutical composition for use according to the eighth aspect of this invention, wherein the immunomodulatory molecule is delivered to target cells in vitro, ex vivo, and/or in vivo, optionally wherein the delivery of the immunomodulatory molecule generates a cell therapy and/or is used as a cell therapy, wherein:
(a) the immunomodulatory molecule is delivered via a lentivirus, an adeno-associated virus (AAV), a lipid nanoparticle (LNP), a viral-like particle (VLP), or electroporation, wherein the immunomodulatory molecule is preferably delivered in form of a nucleic acid, such as a DNA or mRNA, or in form of a protein, optionally wherein the immunomodulatory molecule is permanently or transiently expressed in the target cells after delivery, and/or
(b) a nucleic acid donor template, such as a DNA donor template, containing the immunomodulatory molecule, a gRNA using homology-directed repair (HDR) gene editing, and a lentivirus, a transposase, such as a Sleeping Beauty or a piggyBac transposase, or a CRISPR-nuclease, is delivered to said target cells, and wherein the immunomodulatory molecule is semi-randomly integrated into a target cell via the lentivirus, the transposase, such as the Sleeping Beauty or the piggyBac transposase, or the CRISPR-nuclease, and/or
(c) the immunomodulatory molecule is delivered via a CRISPR-mediated HDR or a prime editing and/or recombinase based approach, such as a PASTE or a PASSIGE based approach, wherein the immunomodulatory molecule is targetedly integrated into a target cell via the CRISPR-mediated HDR or the prime editing and/or recombinase based approach, such as the PASTE or the PASSIGE based approach, wherein, optionally, the immunomodulatory molecule is integrated, such as integrated targetedly, at the locus of the TRAC gene, the TRBC gene, or at the locus of a safe harbor gene.

In one embodiment, which can be combined with each embodiment and/or aspect of this invention, the immunomodulatory molecule is delivered via lentiviral integrated CARs in ex vivo T cells.

In one embodiment, which can be combined with each embodiment and/or aspect of this invention, the immunomodulatory molecule is delivered via LNP/mRNA, which results in the generation of transient CAR-Ts and thereby transient expression of the immunomodulatory molecule.

In one embodiment, which can be combined with each embodiment and/or aspect of this invention, the immunomodulatory molecule, the engineered protein binding domain, the isolated nucleic acid, the product, or the pharmaceutical composition is administered to the subject, such as the patient, by intravenous, intramuscular, subcutaneous, intradermal, intra-peritoneal, and/or intra-pleural injection.

In one embodiment, the immunomodulatory molecule, the engineered protein binding domain, the isolated nucleic acid, the product, or the pharmaceutical composition for use according to the eighth aspect of this invention is administered to a patient by a nucleic acid, such as an mRNA molecule.

An ninth aspect of the present invention, which can be combined with any aspect and/or embodiment of the present invention, pertains to a method of treatment of a patient using a host cell comprising a coding sequence encoding the immunomodulatory molecule according to the first aspect of this invention, or comprising a coding sequence encoding the engineered protein binding domain according to the second aspect of this invention, or the isolated nucleic acid according to the third aspect of this invention, the method comprising the steps of:
(i) obtaining a biological sample from a patient or an allogeneic donor, wherein, preferably the biological sample is a blood sample;
(ii) isolating one or more host cells from said sample; wherein, preferably, the host cell is selected from T cells, natural killer (NK) cells, natural killer T (NKT) cells, lymphocytes, myeloid cells, such as eosinophils, mast cells, basophils, granulocytes, macrophages, and/or any of the abovementioned cells derived from human induced pluripotent stem cells (hiPSCs); preferably T cells, NK cells, and macrophages; more preferably T cells;
(iii) activating and/or stimulating one or more host cells; wherein, optionally, the host cell is a T cell and expansion of one or more host T cells is activated and/or stimulated by contact with an artificial antigen-presenting cell (aAPC); wherein, optionally the aAPC is an aAPC expressing anti-CD28 and anti-CD3 complementarity determining regions (CDRs)
(iv) transfecting the host cells with an expression construct according to the third aspect of this invention;
(v) cultivating the host cells of step (iii) under conditions promoting the expression of the immunomodulatory molecule;
(vi) selecting candidate host cells; wherein preferably the candidate host cells are host cells that constitutively or transiently express the immunomodulatory molecule;
(vii) optionally, performing lymphodepletion on a patient, and
(viii) administering one or more candidate host cells of step (vi) to the patient.

A tenth aspect of the present invention pertains to a T cell, such as a CAR-T cell, comprising a coding sequence encoding an immunomodulatory molecule according to the first aspect of this invention, an engineered protein binding domain according to the second aspect of this invention, or a product obtainable by a method according to the fourth aspect of this invention. This aspect of the present invention also relates to a T cell, such as a CAR-T cell, comprising an isolated nucleic acid according to the third aspect of this invention.

In one embodiment, which can be combined with every other embodiment and/or aspect of this invention, a CAR-T cell further secretes a bispecific molecule, such as a BiTE.

An eleventh aspect of the present invention pertains to a kit comprising a vector containing an expression cassette encoding an immunomodulatory molecule according to the first aspect of this invention, an engineered protein binding domain according to the second aspect of this invention, or a product obtainable by a method according to the fourth aspect of this invention, for transfection of a host cell, optionally, wherein the vector is used to integrate the expression cassette into the genome of the host cell.

In one embodiment, the kit further comprises instructions and/or an instruction manual for its use.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

### BRIEF DESCRIPTION OF THE FIGURES

The figures show:

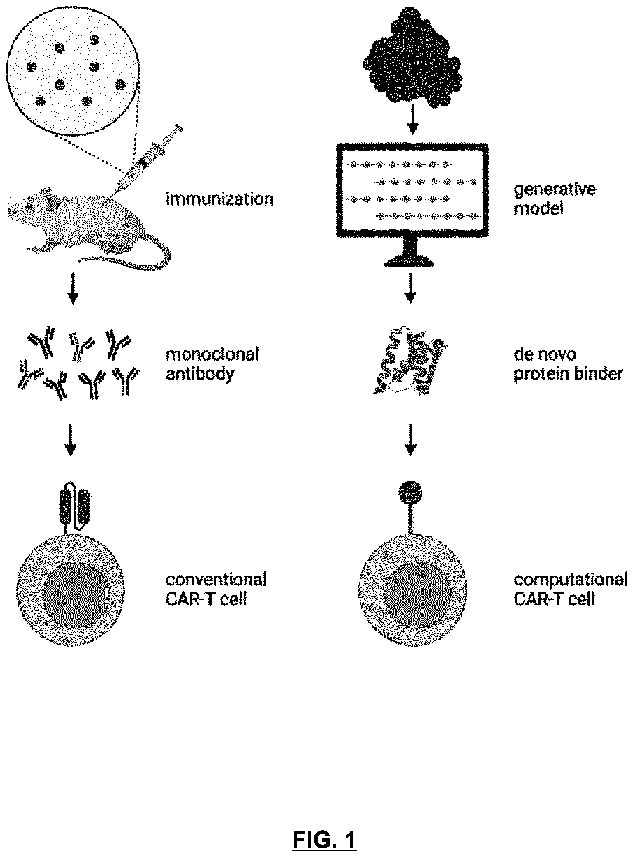

Figure 1 shows the CAR-T cell development process. Left, the current standard method to develop a CAR-T cell therapy to target a specific antigen relies on producing a monoclonal antibody from which the antigen-targeting moiety of the CAR can be derived. Right, the method according to the present invention leverages generative models for protein design. The inventors incorporated the resulting de novo binders into a CAR and engineered it for optimal function in primary human T cells.

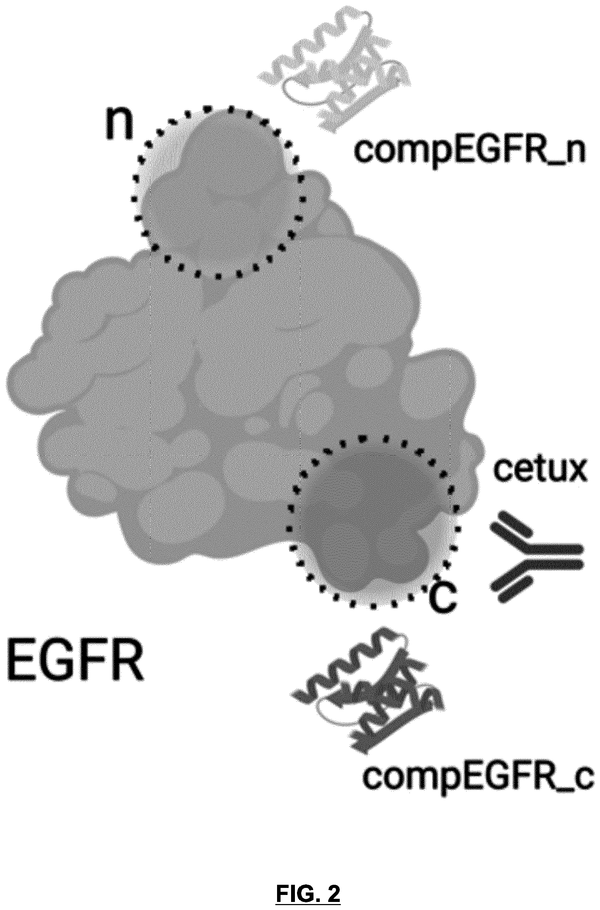

Figure 2 shows the Epithelial Growth Factor Receptor (EGFR) targeting strategy. Mini-protein binders that target domain I (compEGFR_n, yellow) and domain III (compEGFR_c, green) of EGFR, previously described by Cao et al.^{[1]}, were used. As a control, the inventors employed a single-chain variable fragment derived from the monoclonal antibody cetuximab that targets the domain III (cetux, green) of EGFR.

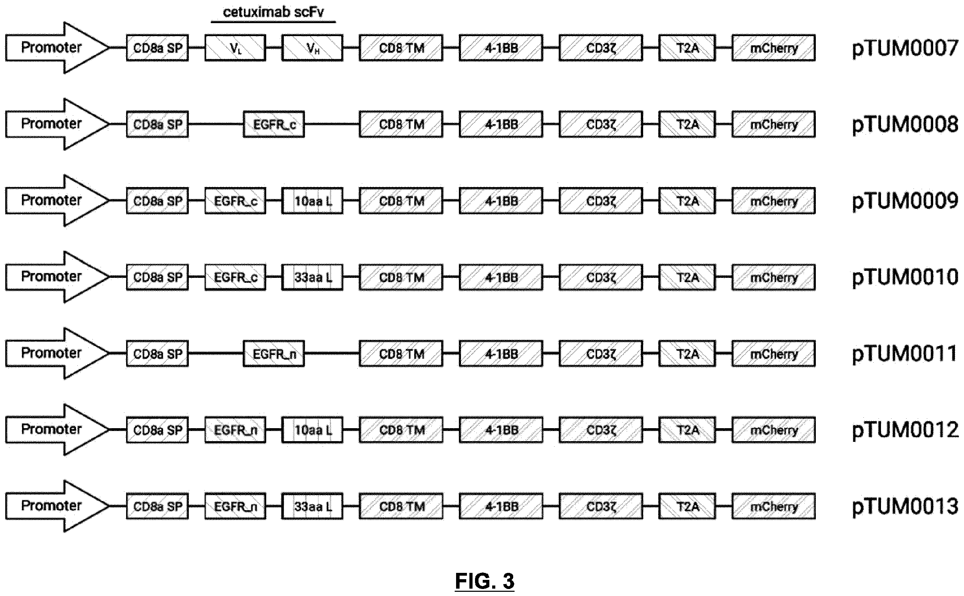

Figure 3 shows the construct designs for cetuxCAR-T (pTUM0007) and compCAR-T (pTUM0008-pTUM0013). All CAR constructs were expressed under the control of an EF1alpha promotor (white), used a CD8 signal peptide (single left dashed), a CD8 hinge/transmembrane domain (double left dashed), a 4-1BB co-stimulatory domain (double left dashed), and a CD3zeta signaling domain (double left dashed). The antigen binding domain (single right dashed) consisted of the light and heavy chain derived from cetuximab (pTUM0007), a de novo mini-binder targeting domain III of EGFR (EGFR_c; pTUMooo8- pTUM00010) or a de novo mini-binder targeting domain I of EGFR (EGFR_n; pTUM0011- pTUM00013). In addition, spacers of different lengths (straight dashed, ten amino acids, or 33 amino acids) were used in some constructs to connect the mini-binder to the hinge/transmembrane domain.

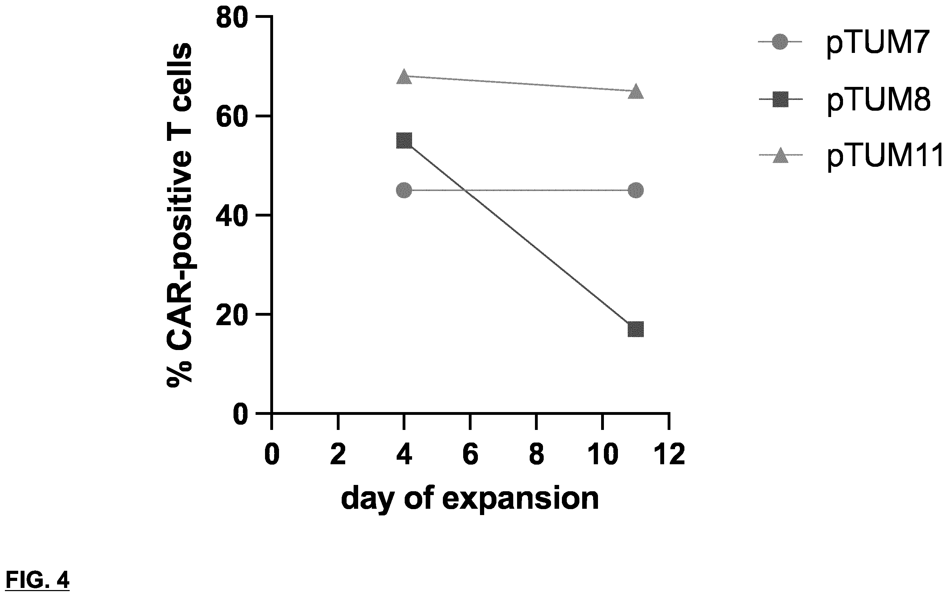

Figure 4 shows the percentage of CAR-positive T cells during cetuxCAR-T (pTUM7) and compCAR-T (pTUM8 and pTUM11) production. Primary human T cells from a healthy donor were transduced with CAR-encoding viral vectors at a multiplicity of infection of 5. The percent of CAR-positive T cells was measured on day 4 and day 11 of the CAR-T production by flow cytometry based on the fluorescent reporter mCherry. Data points show one representative donor.

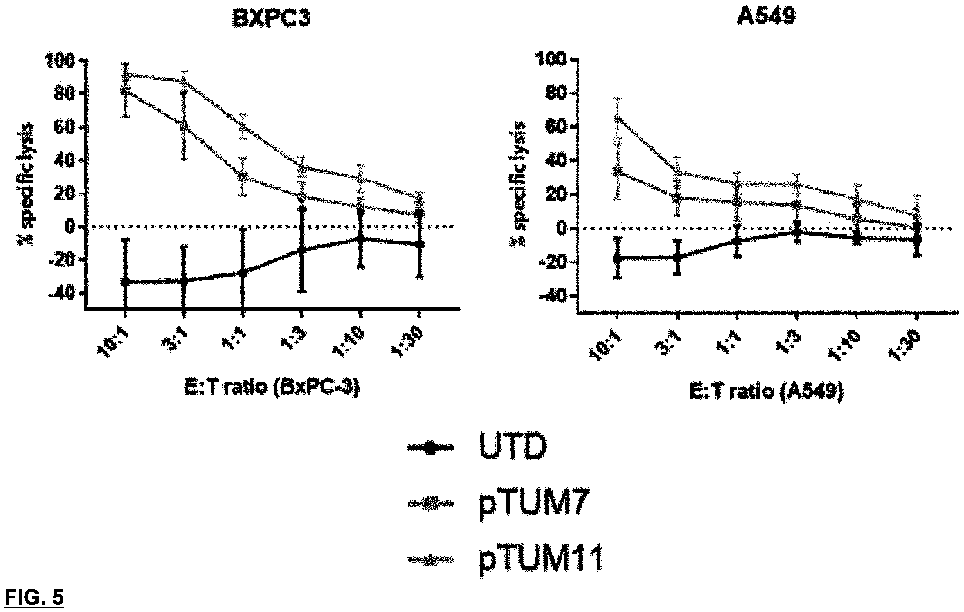

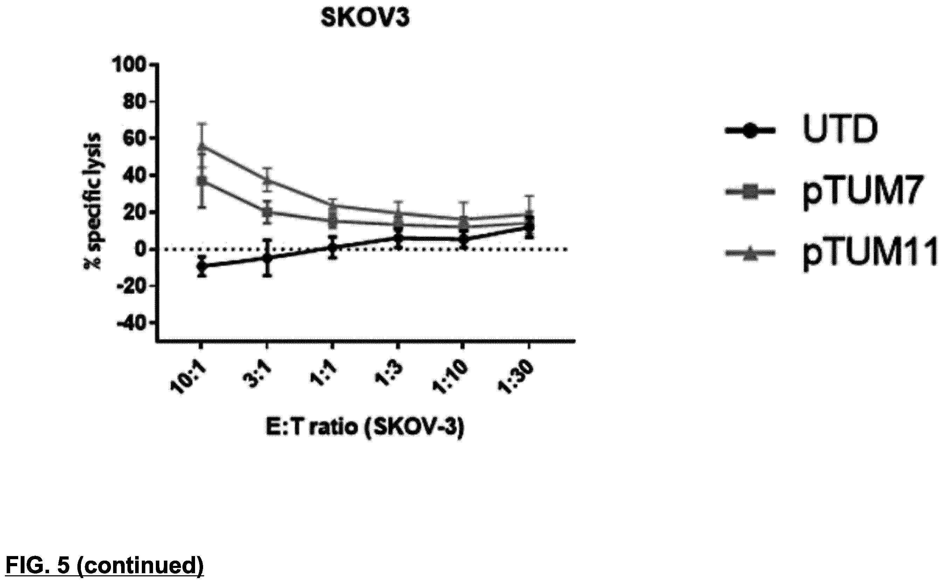

Figure 5 shows the cytotoxic effector function of cetuxCAR-T and compCAR-T. Luciferase-based cytotoxicity assays of cetuxCAR-T (pTUM7) and compCAR-T (pTUM11) against a panel of target cells (BXPC3: human pancreatic cancer, A549: lung cancer SKOV3: ovarian cancer) cocultured at various effector to target ratios (E:T ratios), as indicated on the x-axis. Luciferase activity of targets was measured after 16-18 hours. Data points show the mean ± standard error of triplicates' mean (SEM) from 3 normal donors. UTD refers to "untransduced T cells".

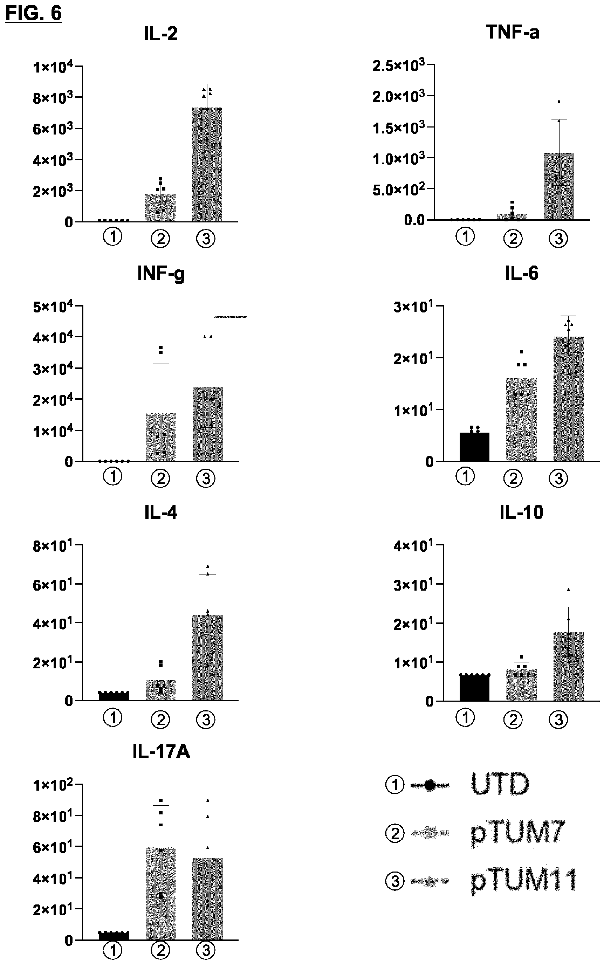

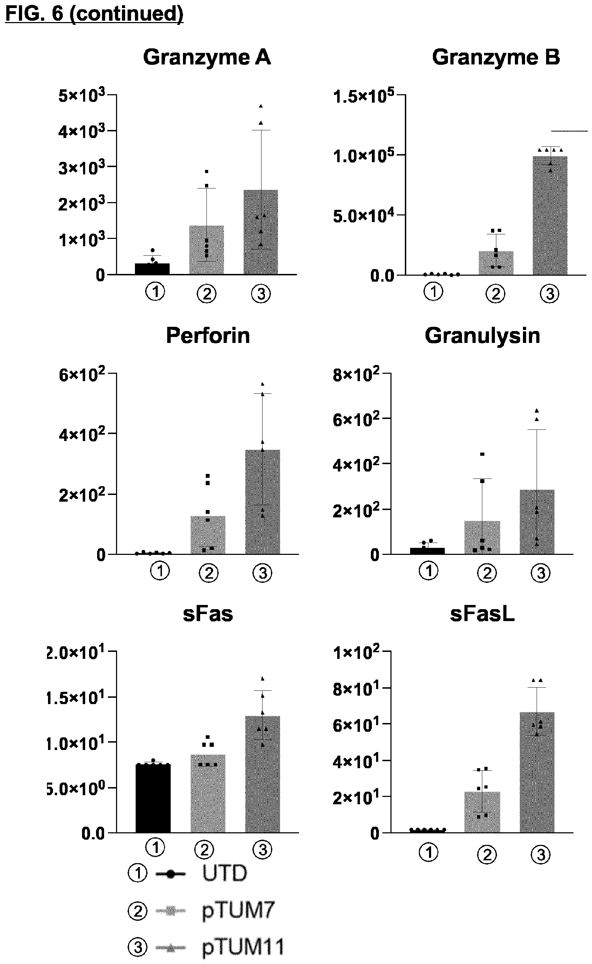

Figure 6 shows Cytokine and lytic protein profiles of cetuxCAR-T and compCAR-T. Levels of cytokines and cytotoxic factors in supernatants of UTDs, cetuxCAR-T (pTUM7), and compCAR-T (pTUM11) after overnight coculture with human BXPC3 pancreatic cancer cells at a 1:1 effector-to-target ratio are shown. Analytes were measured by 13-plex LEGENDPLEX assay in technical duplicates. All concentrations are displayed as picograms per milliliter (pg/mL). Bars show the mean ± SEM of 3 normal donors. UTD refers to "untransduced T cells".

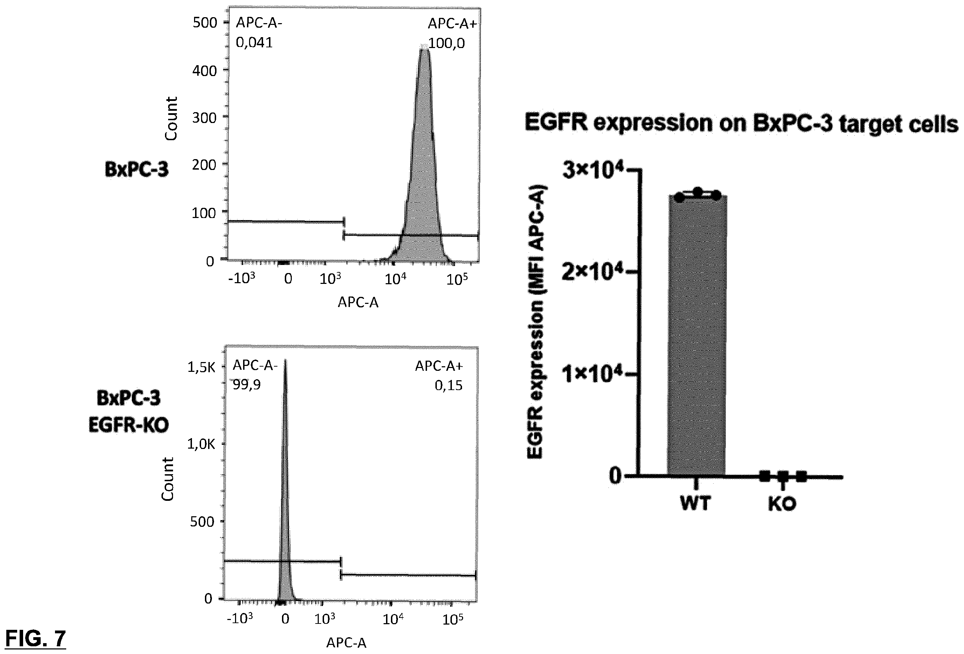

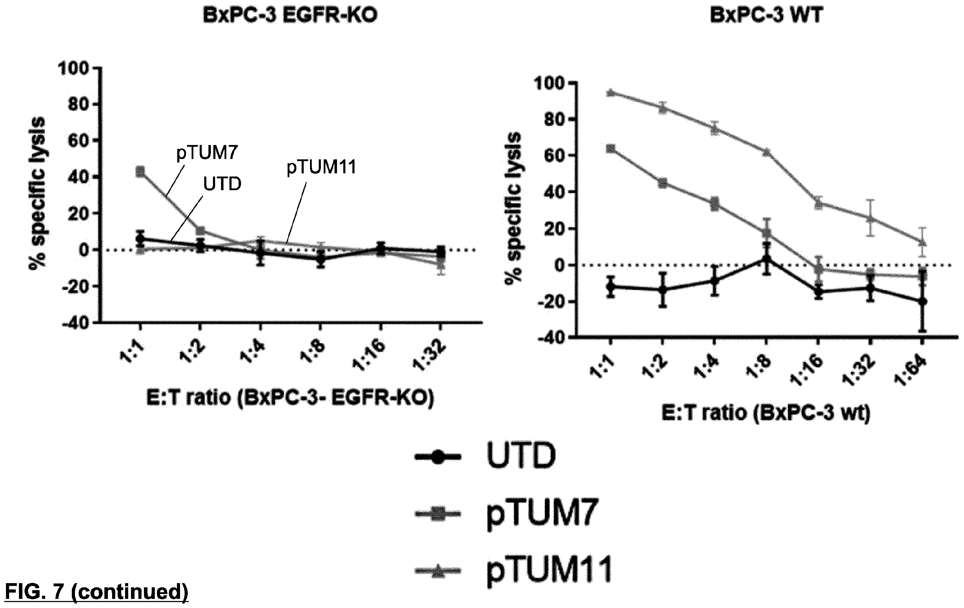

Figure 7 shows the Cytotoxic potential of cetuxCAR-T and compCAR-T against wildtype BXPC3 and EGFR-KO BXPC3 pancreatic cancer cells, a, The inventors used CRISPR-Cas9 gene editing to knock out EGFR from BXPC3 pancreatic cancer cells. Flow cytometry staining confirmed the lack of EGFR expression in knockout (KO) cells, b, BXPC3 EGFR-KO cells and BXPC3 wildtype (wt) cells were used as target cells in a luciferase-based cytotoxicity assay with cetuxCAR-T (pTUM7) and compCAR-T (pTUM11) at various effector to target ratios (E:T ratios), as indicated on the x-axis. Luciferase activity of targets was measured after 16-18 hours of coculture and a subsequent 48-hour recovery period. Compared to BXPC3 wt cells, the vitro cytotoxicity of cetuxCAR-T and compCAR-T against BXPC3 EGFR-KO cells was substantially reduced, indicating antigen-specificity. Data points indicate the mean ± standard error of triplicates' mean (SEM) from 1 normal donor. UTD refers to "untransduced T cells".

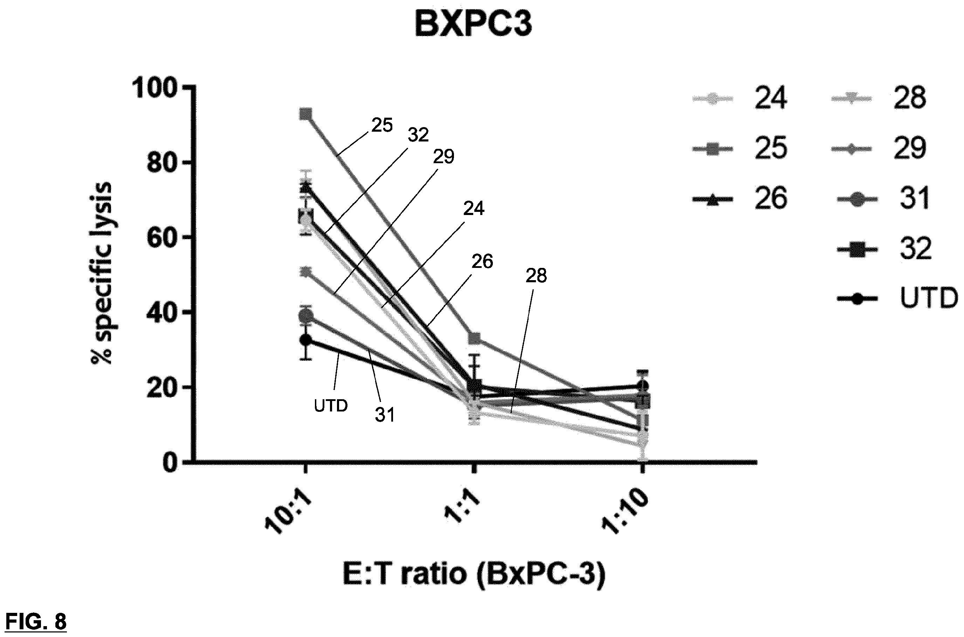

Figure 8 shows the Functional Screening of RFdiffEGFR_c binder CAR-T. From the design campaign for RFdiffusion-based protein binders that target the domain III of EGFR, the inventors chose seven different sequences (listed in Table 1) for experimental screening in a luciferase-based cytotoxicity assay. BXPC3 cells were used as targets and cocultured at various effector-to-target ratios (E:T ratio) with CAR-T cells or UTD, as indicated on the x-axis. Luciferase activity of targets was measured after 16-18 hours. Data points show the mean ± standard error of triplicates' mean (SEM) from 1 normal donor. UTD refers to "untransduced T cells".

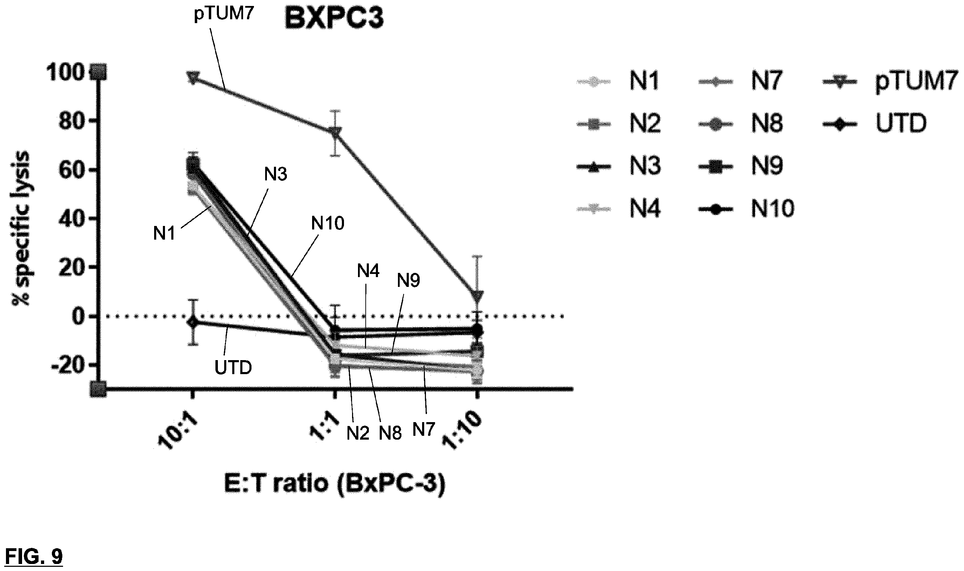

Figure 9 shows the Functional Screening of RFdiffEGFR_n binder CAR-T. From the design campaign for RFdiffusion-based protein binders that target domain I of EGFR, the inventors chose eight different sequences (listed in Table 2) for experimental screening in a luciferase-based cytotoxicity assay. BXPC3 cells were used as targets and cocultured at various effector-to-target ratios (E:T ratio) with CAR-T cells or UTD, as indicated on the x-axis. Luciferase activity of targets was measured after 16-18 hours. Data points show the mean ± standard error of triplicates' mean (SEM) from 1 normal donor. pTUM7: control cetuxCAR-T. UTD refers to "untransduced T cells".

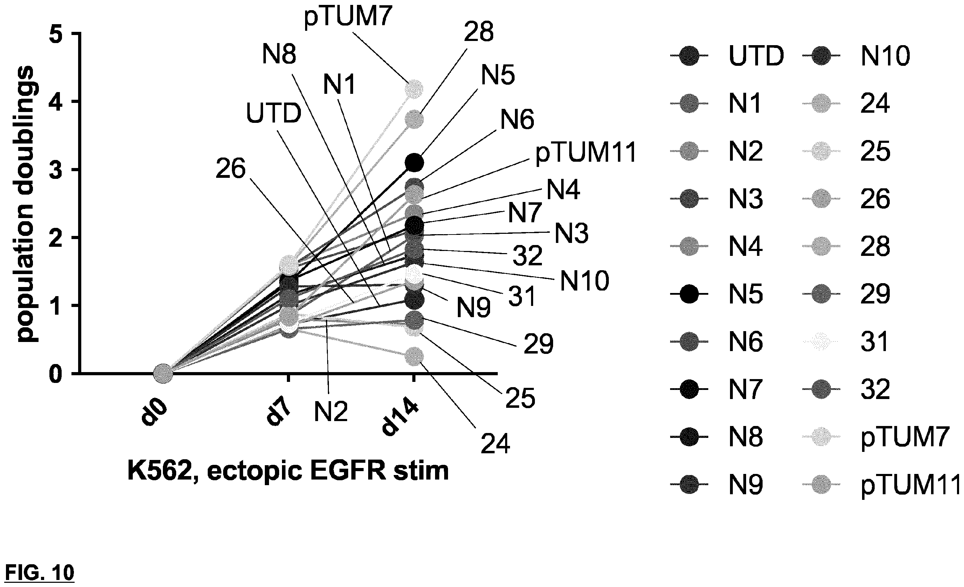

Figure 10 shows the long-term proliferation of RFdiffEGFR binder CAR-T. Growth curves of RFdiffEGFR_c binder CAR-T (24-32) and RFdiffEGFR_n binder CAR-T (N1-N10) in vitro during weekly antigen stimulation with irradiated K562 cells expressing EGFR. Results are displayed as population doublings of 1 normal donor. pTUM7: control cetuxCAR-T. UTD refers to "untransduced T cells".

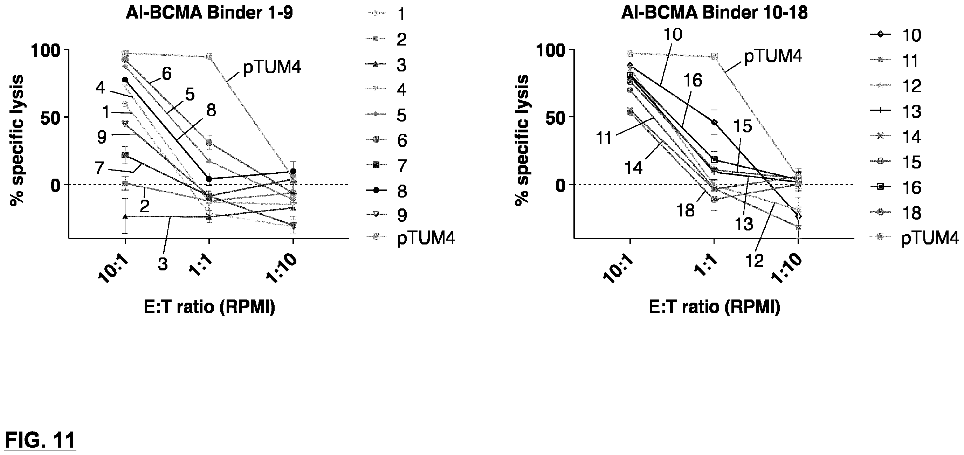

Figure 11 shows that Functional Screening of RFdiffBCMA binder CAR-T. From the design campaign for RFdiffusion-based protein binders that target BCMAR, we chose 17 different sequences (listed in Table 3) for experimental screening in a luciferase-based cytotoxicity assay. RPMI-8226 cells with endogenous BCMA surface expression were used as targets and cocultured at various effector-to-target ratios (E:T ratio) with CAR-T cells or UTD, as indicated on the x-axis. Luciferase activity of targets was measured after 16-18 hours. Data points show the mean ± standard error of triplicates' mean (SEM) from 1 normal donor. pTUM4: control BCMA-CAR-T using an scFv-based binding domain. UTD refers to "untransduced T cells".

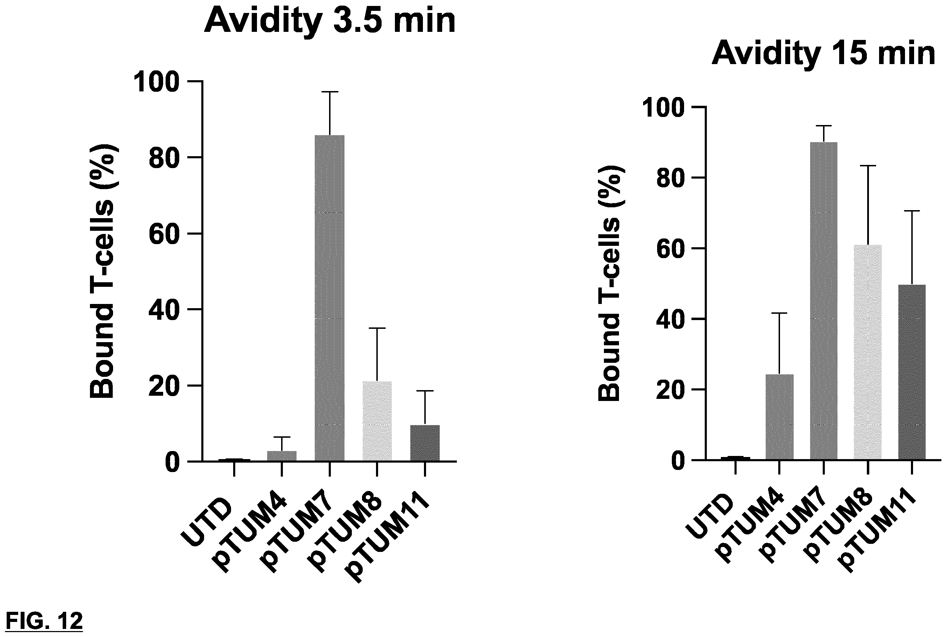

Figure 12 shows Immune synapse binding avidity to SK-OV-3 cells measured by acoustic force microscopy (z-Movi assay). SK-OV-3 cells that endogenously express EGFR were plated as monolayer and co-incubated with UTDs, cetuxCAR-T (pTUM7), and compCAR-T (pTUM8 and pTUM11) for 3,5 minutes and 15 minutes. Increasing acoustic force was applied, and the percentage of bound cells was measured. Data represent the percentage of bound T cells at an applied force of 1000 pico-newton as mean ± SD from three microfluidic chips.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Computational CAR binder design and production of CAR-T cells

To test the concept of using de novo-generated protein binders (i.e., immunomodulatory molecules comprising an engineered protein binding domain according to the present invention) as antigen-targeting moiety, the inventors first sought to design CARs that employ a previously described binder that has been experimentally validated. The inventors chose the mini-protein binders Cao and colleagues described that bind to the C- and N-terminal domains of the epithelial growth factor receptor (EGFR)^{[1]}. These de novo binders are 63 and 62 amino acids long and have been experimentally optimized. Based on these binders, the inventors designed second-generation CARs, employing a 4-1BB co-stimulatory domain and a CD3zeta signaling domain.

To design CARs that incorporate computationally designed binders ("compCAR") targeting EGFR, the inventors leveraged mini-binder proteins for the domain I (compEGFR_n) and domain III (compEGFR_c) described by Cao and colleagues ^{[1]} (Figure 2). Since the optimal cell-to-cell distance for the compCAR to engage the epitopes in domain I and domain III of EGFR is unclear, the inventors used spacers of different lengths (no spacer, 10 amino acid spacer, and 33 amino acid spacer) to connect the compEGFR_n and compEGFR_c binders to canonical CAR structures (Figure 3). Expressing the EGFR-targeting compCARs in T cells resulted in variable yields of CAR-T cells (compCAR-T). The design using the compEGFR_c binder without a spacer yielded very few CAR-positive T cells, making a thorough characterization of the product impossible (Figure 4). In parallel, a scFv-based anti-EGFR CAR based on the monoclonal antibody cetuximab (cetuxCAR) with identical transmembrane and intracellular domains was used as a control.

In one exemplary embodiment, the inventors found that a computational protein binder EGFR_n was 3.5-fold lighter and had a coding sequence that is 3.8-fold shorter compared to the cetuximab-derived scFv (7.64 kDa versus 25.8 kDa and 186 bp versus 723 bp, respectively).

### Example 2: Functional testing of computational CAR-T cells

The inventors then set out to characterize CAR-T cells incorporating such computational protein binders compared to a canonical CAR design that employs a scFv-binding domain derived from a monoclonal antibody. The inventors characterized the binding and functional properties of the engineered CARs when transduced into primary human T cells (compCAR-T and cetuxCAR-T). As shown in Figure 5, CompCAR-T lyse human pancreatic, lung, and ovarian cancer cell lines that endogenously express EGFR at levels in a similar way as cetuxCAR-T. Subsequently the inventors analysed the supernatants from cocultures of compCAR-T and cetuxCAR-T with EGFR-expressing target cells, which demonstrated antigen-specific production of Th1-type cytokines and apoptotic factors at comparable rates (Figure 6).

Next, the inventors generated cell line counterparts in which the EGFR gene was knocked out. The lack of EGFR expression was confirmed by surface staining and subsequent flow cytometry analysis. Notably, lysis of the cell lines lacking EGFR expression was substantially reduced for compCAR-T and cetuxCAR-T. For compCAR-T, the residual cytotoxicity activity was even from untransduced control T cells (UTD), consistent with antigen-specific cytotoxicity (Figure 7).

The inventors proceeded to test the binding strength of the CARs generated according to the present invention to EGFR-expressing target cells. Each CAR was transduced into Jurkat T cells at a multiplicity of infection of 0.5 to eliminate differences in CAR expression levels. CAR-expressing Jurkat cells were sorted to have matching mean fluorescence intensities. The binding avidity of the immune synapse that compCAR-T cells and cetuxCAR-T cells formed with EGFR-expressing target cells was measured by acoustic force microscopy. The inventors found that compCAR bound their targets with a faster off-rate compared to cetuxCAR (Figure 12).

**Table 1 | Designed RFdiffEGFR_c binders**

| **Short name (****Fig. 8****)** | **Alternative name** | **Amino acid sequence** | **SEQ ID NO:** |
|---|---|---|---|
| 24 | RFdiffEGFR _c1 | SEKMKNIQELLAEADKYLEKAKAAKTKEEAK AYLRKAAEKLLQSPLSGLQEVASRILEKLEKE | 3 |
| 25 | RFdiffEGFR _c2 | MKSKLKSYSEAALLYLTGGKEKLELAEKLLSE MYDLLGDEAAKERAKKNIEELRKEEEEKLKK | 4 |
| 26 | RFdiffEGFR _c3 | MEEEKETSEYMRQTLHETLVAMGLDELALE LYSEAKEKGWNLQEIVDMNDKRLAAIAAEEA AA | 5 |
| 27 | RFdiffEGFR _c4 | GDRNIARMMNFIKEKLAELRADPAGAPKAE AIEKRMEEISKALKEDFSTGLVLLNELYKELL | 6 |
| | | D | |
| 28 | RFdiffEGFR _c5 | MENLINEYKNAADELYKKALEKNPDLAFEII MEVIDLEEKKETVTEEDIKEKIKKLKERLALL | 7 |
| 29 | RFdiffEGFR _c6 | LEEKLKEAEKKLKETIKSVGVSQETLDEINNII KSQHAKLALNGVGEGEYVKVLNELIEKIKK | 8 |
| 30 | RFdiffEGFR _c7 | DLMEQIARSLRDEFVARLEAEGLGPELIAEA RERLEKLLSVLEASTLKLLLAEVEEWVARKL A | 9 |
| 31 | RFdiffEGFR _c8 | MREEELKKRREEVLKKVEELTKLGGKDLEFK AELAELLEKVEEGDEEALKELDRLLDEAFKK L | 10 |
| 32 | RFdiffEGFR _c9 | SIPSELQLLALKLLDLTDKLAKDPSVAKEAKE LRDKIIEAIRSGDEKELLEIKKELDAFLASK | 11 |
| 33 | RFdiffEGFR _c10 | SDATRQLESLLDQIEALLAAGDLEAATQQWQ QLVALLGDLSAEDLEKLLPRIQAAQQAFMAL P | 12 |

**Table 2 | Designed RFdiffEGFR_n binders**

| **Short name (****Fig. 8****)** | **Alternative name** | **Amino acid sequence** | **SEQ ID NO:** |
|---|---|---|---|
| N1 | RFdiffEGFR _n1 | MDKEKEIEEQAKKLKEIAEKLKKELGADESE VNLIVELAVKRIELGMSFEDALKEAIEQLKK | **13** |
| N2 | RFdiffEGFR _n2 | NKAEEEREKLKEEIKEIIEELIKKNPSLTQETI EKINSTLENLILAGASKEDIIKALKKEIE | **14** |
| N3 | RFdiffEGFR _n3 | ADELQEKLNKLNALMLKYRNSRATEIYYNLA LKLEELAKTGTEETKEKVKKAEEKLKEYEAK | **15** |
| N4 | RFdiffEGFR _n4 | SSAEYQALLEQKRLEARRLLAEALLKGSEELK EKLKEKLKEWREEGFPEELIKELEEELEKA | **16** |
| N5 | RFdiffEGFR _n5 | LLEEFKKELDELLEKIKSGEVTEEDLLKKTEE AIEEVKKLPNAEKYQTELKRLVAEALLKLS | **17** |
| N6 | RFdiffEGFR _n6 | EEEEQKKNLLKQLKIKAEFLIEKTGDKEKVL ELEKKYIEALDSGKNKEEAEKLLKEMESLE | **18** |
| N7 | RFdiffEGFR _n7 | VELKREATLLELEAIEAAKAGDEEKRDELIKK LVELMKKLGLPQEQIDSKVEFIKNLAKKTK | **19** |
| N8 | RFdiffEGFR _n8 | AKEAEEKAKKLEELIKEVEEELRKMAKEGKS EKELLDYANKKIPEKPAEATILVLKIVEEEK | **20** |
| N9 | RFdiffEGFR _n9 | SEKVLEAKKKILNLESEISKKLGKLPEEVKELL KKTWEALEKDIEEAEKLIEEIKKKVEEIL | **21** |
| N10 | RFdiffEGFR _n10 | KEEELKEFMEELREILEKKGLPEEEIEELLKK YKEELEKLIKSKNETEVEIFKAIKRQEALS | **22** |

**Table 3 | Designed RFdiffBCMA binders**

| **Alternative name** | **Amino acid sequence** | **SEQ ID NO:** |
|---|---|---|
| RFdiffBCMA _1 | SSENEKLLELFLNFYEKNVGTLTKEQKEHILKLAKETLNE ELYKIFKEKMEEIEKKYKEEKA | **23** |
| RFdiffBCMA _2 | SSSKLEELKRVKEAIAKALEKALKTNPELAARLQEILDVL EEYLEAGKLDEAWKELGALLGS | **24** |
| RFdiffBCMA _3 | MEEKRKQLERGLAALEAAIEAGDKETAELYLRYIEYLAS QDEELKAYVEARFAANPALKAYL | **25** |
| RFdiffBCMA _4 | SSLQALTERLFNEALELKEELKSLSPEEREKLIKERLEEV KKEDPENYPLFSRIVESMYKSL | **26** |
| RFdiffBCMA _5 | SSLRERAYRTLREAAAATPESERPAVAARLAELWERLSE ENIEAVLAEAEALLAEQLARAAA | **27** |
| RFdiffBCMA _6 | STTTEQALAILARVAELVAKGLTPEDRESLAAARALAESV IASSSPEDAAKLRAALAELEAG | **28** |
| RFdiffBCMA _7 | SEELLELVKKYMKERIEKAPLEKLPEVLEKLKLLAERIGL SEEDIKKLMEEAKALIAKKKAA | **29** |
| RFdiffBCMA _8 | SAELEAIKARIRAEWDALGDEEGKEVMEKIFEYLLKNFP ELTPEEIEQLLEQVRQSILAQRA | **30** |
| RFdiffBCMA _9 | SSLEQREKEFFEELDAMIAAGASLEELKAKVASFLRENPE ISSETYNKLRARVKAYQAALAA | **31** |
| RFdiffBCMA _10 | MSDEEIEALVERIVASAQGLTEEQKAQLKAFLLELFKQRS SLSNSEILSLIREKVEALKAAA | **32** |
| RFdiffBCMA _11 | SAAAARRAELVQKIIDYLQKTLKALGKLEEAPKVIAAVQA LVEAGASELELLRYAYQVREAL | **33** |
| RFdiffBCMA _12 | MTLAEIEAERQALLAEIREEGLSLAEAQKRILDWLLELYE RTHNPELVSQLARDFVAALEAL | **34** |
| RFdiffBCMA _13 | RKFVTPESLKKYIEKNREKYEEVKKYMEALKKAGYMTPE VWKLFDELFEKIEKERELEKLLK | **35** |
| RFdiffBCMA _14 | SAAQAALAAELLARIEALRAEGASLEELAELLDEVRKALP LLTPEQQAEVIAAVRALLESFK | **36** |
| RFdiffBCMA _15 | SPEEERRRLQERLESYAAYLEAQIAAGVDPETRARYEAAL AIIRESEDPEEAVEKLREFNAS | **37** |
| RFdiffBCMA _16 | SAAARRALLEARLAELEENLSKLPPEQAEQVLAYLKKSYP SMPPETIERFEAIIEQKKAELA | **38** |
| RFdiffBCMA _17 | SAELEQLRARALERAQTLSVEEIEELRTTLLALLRYRGAP EEEIAAVEAEFEAIIAKRAAE | **39** |
| RFdiffBCMA _18 | SGAARAELIDAILREAQELLEAGAPLAEVEAWLRERAAA AGLSPETTAALLAWVRRRYEELA | **40** |

### Example 3: RFdiffusion-based CAR binder design and production of CAR-T cells

Having established the general feasibility of using small computational protein binders as antigen-targeting moieties in CARs and their functionality when expressed in human T cells, the inventors designed new protein binders using the AI-based RFdiffusion tool. The inventors performed design campaigns for proteins that bind domain I of EGFR (RFdiffEGFR_n), domain III of EGFR (RFdiffEGFR_c), and the extracellular domain of the B-cell maturation antigen (BCMA). Functional screening of the RFdiffEGFR_c- and RFdiffEGFR_n-based CAR-T showed varying cytotoxic activity and long-term proliferation against EGFR-expressing targets (Figure 8 9, and 10). Functional screening of the RFdiff BCMA binder based CAR-T showed varying cytotoxic activity (Figure 11).

Importantly, the inventors found that computationally generated binders according to the present invention do not automatically work in CAR-T cells. Even validation of computationally generated binders according to the present invention in short term toxicity assays, Z-movie avidity, and long-term proliferation assays showed highly various results regarding binder efficiencies from both computational (Example 1) and/or AI-generated (RFdiffusion, Example 3) origin. In sum, activity in any of these assays did not predict activity in another assay, indicating that the validation of binders is a highly complex process. Accordingly, it is non-obvious whether a computationally generated binder works in CAR-T cells or not, and many different non-obvious experimental tests are necessary for determining whether a computationally generated binder works in CAR-T cells or not.

### REFERENCES

The references are:
[1] Cao, L. et al. Design of protein-binding proteins from the target structure alone. Nature 605, 551-560 (2022). https://doi.org:10.1038/s41586-022-04654-9
[2] Bennett, N. et al. Improving de novo Protein Binder Design with Deep Learning. Nature Communications, 14, Article number: 2625 (2023) https://www.nature.com/articles/s41467-023-38328-5
[3] Watson, J. et al. De novo design of protein structure and function with Rfdiffusion. Nature 620(7976):1089-1100 (2023). https://doi.org/10.1038/s41586-023-06415-8
[4] Mansilla-Soto, J. et al. HLA-independent T cell receptors for targeting tumors with low antigen density. Nat Med. 2022 Feb;28(2):345-352. https://doi.org/10.1038/s41591-021-01621-1.

## Claims

1. An immunomodulatory molecule, comprising an engineered protein binding domain, a linker and/or a hinge-region, and a domain for the activation and/or stimulation of an immune effector cell;
wherein the engineered protein binding domain comprises two or three alpha-helices and has a length of 59-100 amino acids, preferably of 60-90 amino acids, even more preferably of 60-80 amino acids, even more preferably of 60-70 amino acids, and most preferably of 60-65 amino acids;
wherein, optionally, the engineered protein binding domain contains one or more beta-sheet structures;
wherein the engineered protein binding domain is not derived from an animal or a human; wherein the engineered protein binding domain is not derived from a natural protein binding molecule, such as an antibody or a DARPin; wherein the engineered protein binding domain is not the result of a ribosome/phage display technique; and wherein the engineered protein binding domain is not an affibody.

2. The immunomodulatory molecule according to claim 1, wherein the domain for the activation and/or stimulation of an immune effector cell is an intracellular domain for the activation and/or stimulation of the immune effector cell; or wherein the domain for the activation and/or stimulation of an immune effector cell is a domain binding to an immune effector cell and thereby activating and/or stimulating the immune effector cell; and/or wherein the immune effector cell is selected from a group comprising T cells, natural killer (NK) cells, natural killer T (NKT) cells, lymphocytes, myeloid cells, such as eosinophils, mast cells, basophils, granulocytes, macrophages, and any of the abovementioned cells derived from human induced pluripotent stem cells (hiPSCs); wherein, preferably, the immune effector cell is a T cell, an NK cell, or a macrophage; wherein, more preferably, the immune effector cell is a T cell.

3. The immunomodulatory molecule according to claim 1 or 2, wherein the engineered protein binding domain binds to an antigen selected from a group comprising tumor-associated antigens, tumor-specific antigens, antigens associated with a disease, antigens associated with toxin removal, and antigens associated with organ transplant rejection; wherein, preferably, the engineered protein binding domain binds to a tumor-associated antigen;
wherein, preferably, the tumor-associated antigens are selected from a group comprising: Epidermal growth factor receptor (EGFR), B-cell maturation antigen (BCMA, TNFRSF17), CD3, CD5, CD7, B-lymphocyte antigen CD19 (CD19), B-lymphocyte antigen CD20 (CD20), CD22, CD37, CAMPATH-1 antigen (CD52), CD70, CD79b, epidermal growth factor receptor variant III (EGFRvIII), Fibroblast activation protein (FAP), Fibroblast activation protein alpha (FAPalpha), G-protein coupled receptor family C group 5 member D (GPRC5D), Receptor tyrosine-protein kinase erbB-2 (HER2, ERBB2), Interleukin-13 receptor subunit alpha-2 (IL-13Ra2, CD213A2), Mesothelin (MSLN), Mucin short variant S1 (MUC1), Mucin-16 (MUC16, CA-125), prostate-specific membrane antigen (PSMA, GCPII), Prostate stem cell antigen (PSCA), Tyrosine-protein kinase transmembrane receptor ROR1 (ROR1, NTRKR1), SLAM family member 7 (SLAMF7, CD319), Transmembrane activator and CAML interactor (TACI, TNFRSF13B), and Vascular endothelial growth factor (VEGF); more preferably EGFR, BCMA, TACI, CD19, CD3, FAP, and HER2; even more preferably EGFR, BCMA, TACI, CD19, and CD3; and most preferably EGFR and BCMA;
wherein, preferably, the engineered protein binding domain binds to a cell surface antigen;
wherein, preferably, the tumor-specific antigen is epidermal growth factor receptor variant III (EGFRvIII); and
wherein, preferably, the antigens associated with a disease are antigens associated with an autoimmune disease, an infectious disease, a cardiovascular disease, a cerebrovascular disease, a neurodegenerative disease, a dermatological disease, a genetic disease, an aging-related disease, a nephrological disease, an endocrinological disease, a fibrotic disease, a metabolic disease, an obesity-related disease, a gynecological disease, an ophthalmic disease, an otorhinolaryngologic disease, an allergy, and/or an immune deficiency.

4. The immunomodulatory molecule according to any of the foregoing claims, wherein a binding kinetic of a binding of the engineered protein binding domain to a target of the engineered protein binding domain is different from a binding kinetic of a binding of an antibody-derived single-chain variable fragment (scFv) binding domain to the same target; wherein, preferably, the binding kinetic of the engineered protein binding domain is slower or faster than the binding kinetic of the scFv binding domain; wherein, more preferably, the binding kinetic of the engineered protein binding domain is slower than the binding kinetic of the scFv binding domain; and/or
wherein a binding avidity and/or affinity of a binding of the engineered protein binding domain to a target of the engineered protein binding domain is different from a binding avidity and/or affinity of a binding of an scFv binding domain to the same target; wherein, preferably, the binding avidity and/or affinity of the engineered protein binding domain is lower or higher than the binding avidity and/or affinity of the scFv binding domain; wherein, more preferably, the binding avidity and/or affinity of the engineered protein binding domain is lower than the binding avidity and/or affinity of the scFv binding domain; and/or wherein a cytotoxicity of the immunomodulatory molecule is lower than a cytotoxicity of an immunomodulatory molecule containing one or more scFv binding domains directed at the same target as the engineered protein binding domain of the immunomodulatory molecule.

5. The immunomodulatory molecule according to any of the foregoing claims, wherein the immunomodulatory molecule is a chimeric antigen receptor (CAR), a HLA-independent T cell receptor (HIT), or a bispecific molecule, such as a bispecific T cell engager (BiTE); wherein, optionally the immunomodulatory molecule contains modifications in the CAR, the HIT, or the bispecific molecule to modulate the potency of an activation and/or stimulation of an immune effector cell, such as to reduce T cell exhaustion.

6. The immunomodulatory molecule according to claim 5, wherein the immunomodulatory molecule is a CAR, and the CAR comprises:
(a) an engineered protein binding domain comprising two or three alpha-helices and 59-100 amino acids; preferably 60-90 amino acids; even more preferably 60-80 amino acids, even more preferably 60-70 amino acids; most preferably 60-65 amino acids; wherein, optionally, the engineered protein binding domain contains one or more beta-sheet structures or wherein the engineered protein binding domain contains no beta-sheet structures;
(b) a transmembrane domain;
(c) a hinge-region connecting the (a) engineered protein binding domain and the (b) transmembrane domain;
(d) optionally, a linker connecting the (a) engineered protein binding domain and the (c) hinge-region; and
(e) a domain for the activation and/or stimulation of an immune effector cell, wherein the domain for the activation and/or stimulation of an immune effector cell is an intracellular domain that activates and/or stimulates the immune effector cell upon binding of the engineered protein binding domain to a target region.

7. The immunomodulatory molecule according to claim 5 or 6, wherein the immunomodulatory molecule is a CAR, and wherein:
(i) the hinge-region has a length of 10 to 200 amino acids, preferably of 20 to 100 amino acids;
wherein, optionally, the hinge-region comprises an amino acid sequence having a sequence identity of at least 70%, preferably at least 80%, more preferably at least 90%, and most preferably at least 95% sequence identity, to an amino acid sequence selected from SEQ ID NOs: 46 to 49, or, in each case independently, optionally with no more than ten, nine, eight, seven, six, five, four, preferably no more than three, two or one, amino acid substitution(s), insertion(s) or deletion(s) compared to these sequences, and/or
(ii) the linker has a length of 0 to 100 amino acids; preferably 0 to 33 amino acids; wherein, optionally, the linker has one or more rigid and/or flexible segments;
wherein, optionally, the linker comprises an amino acid sequence having a sequence identity of at least 70%, preferably at least 80%, more preferably at least 90%, and most preferably at least 95% sequence identity, to an amino acid sequence selected from SEQ ID NOs: 41,42, and 91, or to an amino acid sequence encoded by the nucleotide sequence selected from SEQ ID NOs: 43, 44, and 92, or, in each case independently, optionally with no more than ten, nine, eight, seven, six, five, four, preferably no more than three, two or one, amino acid substitution(s), insertion(s) or deletion(s) compared to these sequences, and/or
(iii) the transmembrane domain (TM) is selected from a group comprising the TMs of CD8, CD28, an alpha, beta or zeta chain of a T cell receptor, CD3 epsilon, CD45, CD4, CD5, CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRFI), CD160, CD19, IL2R beta, IL2R gamma, IL7R , ITGA1 (CD49a, VLA1), ITGA4 (IA4, CD49D), ITGA6(VLA-6)F, CD49, ITGAD, CD1 1d, ITGAE, CD103, ITGAL, CDI la, LFA-1, ITGAM, CDI Ib, ITGAX, CDI Ic, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), SLAMF6 (NTB-A, Lylo8), SLAM (SLAMF1, CD150, IPO-3), BLAME (SLAMF8), SELPLG (CD162), LTBR, PAG/Cbp, NKp44, NKp30, NKP46, NKG2D, and/or NKG2C; preferably the TM of CD8 or CD28; more preferably the TM of CD8.

8. The immunomodulatory molecule according to any one of claims 5 to 7, wherein the immunomodulatory molecule is a CAR, and wherein the intracellular domain that activates and/or stimulates the immune effector cell upon binding of the engineered protein binding domain to a target region comprises a signaling domain selected from a group comprising the signaling domains of CD3 zeta, TCR zeta, FcR gamma, FcR beta, CD3 gamma, CD3 theta, CD3 delta, CD3 epsilon, CD22, CD79a, CD79b, and CD66d; preferably the signaling domains of CD3 zeta; and
wherein, optionally, the intracellular domain that activates and/or stimulates the immune effector cell upon binding of the engineered protein binding domain to a target region further comprises one or more co-stimulatory domains; wherein, preferably, the one or more co-stimulatory domains is/are selected from a group comprising the co-stimulatory domains of 4-1BB (CD137), CD28, CARD11, CD2, CD7, CD27, CD30, CD40, CD54 (ICAM), CD83, CD134 (OX40), CD150 (SLAMF1), CD152 (CTLA4), CD223 (LAG3), CD270 (HVEM), CD273 (PD-L2), CD274 (PD-L1), CD278 (ICOS), DAP10, LAT, NKD2C SLP76, TRIM, and ZAP70; preferably selected from the co-stimulatory domains of 4-1BB (CD137) and CD28; wherein, even more preferably the co-stimulatory domain is the co-stimulatory domain of 4-1BB (CD137).

9. The immunomodulatory molecule according to claim 5, wherein the immunomodulatory molecule is a bispecific molecule, such as a bispecific T cell engager (BiTE), and wherein the bispecific molecule comprises at least two binding domains,
wherein the first binding domain is an engineered protein binding domain comprising two or three alpha-helices and 59-100 amino acids; preferably 60-90 amino acids; even more preferably 60-80 amino acids, even more preferably 60-70 amino acids; most preferably 60-65 amino acids; wherein, optionally, the engineered protein binding domain contains one or more beta-sheet structures or wherein the engineered protein binding domain contains no beta-sheet structures; and
wherein the second binding domain is a domain for the activation and/or stimulation of an immune effector cell by specifically binding to an immune effector cell and thereby activating and/or stimulating the immune effector cell; and
wherein the linker and/or the hinge-region has a length of 5 to 200 amino acids; wherein optionally, the linker and/or the hinge-region has one or more rigid and/or flexible segments.

10. The immunomodulatory molecule according to any one of claims 5 to 9, wherein the immunomodulatory molecule comprises an engineered protein binding domain targeting and/or binding to EGFR and/or BCMA, optionally wherein the engineered protein binding domain comprises an amino acid sequence according to any one of SEQ ID NOs: 3 to 40.

11. An engineered protein binding domain, wherein said engineered protein binding domain is removed from an immunomodulatory molecule according to claim 10, and wherein said engineered protein binding domain is used outside of the immunomodulatory molecule, optionally wherein said engineered protein binding domain is fused to an Fc-part or a CH3 domain of an antibody, and/or wherein said engineered protein binding domain is used as a binder drug conjugate.

12. An isolated nucleic acid comprising a sequence encoding for an immunomodulatory molecule according to any of claims 1 to 10, or encoding for an engineered protein binding domain according to claim 11, or an expression construct for expressing the immunomodulatory molecule according to any of the foregoing claims in a (host) cell, preferably further comprising a promoter and/or a terminator sequence.

13. A method of obtaining a chimeric antigen receptor (CAR) expressing immune effector cell product, such as a CAR-T cell product, and/or a HLA-independent T cell receptor (HIT) expressing immune effector cell product, such as a HIT-T cell product, and/or a bispecific molecule expressing immune effector cell product, such as a bispecific T cell engager (BiTE) expressing immune effector cell product, comprising an immunomodulatory molecule, and/or a bispecific molecule, such as a bispecific T cell engager (BiTE), comprising an engineered protein binding domain; wherein, optionally, the immunomodulatory molecule is the immunomodulatory molecule according to any of the claims 1 to 10, and/or the engineered protein binding domain is as defined in any one of claims 1 to 10; wherein the method comprises the steps:
i) identifying a target region in a target protein;
ii) running a computer program comprising instructions for implementing a method of identifying an engineered protein binding domain to the target region in the target protein, wherein the identification is a structure-based, machine learning, and/or diffusion-based identification method, wherein the step of running the computer program (ii) is repeated for a number of times, such as at least 10 times, at least 20 times, at least 30 times, at least 40 times, at least 50 times, at least 60 times, at least 70 times, at least 80 times, at least 90 times, or any number of times, preferably wherein the step of running the program is repeated at least 50 times, thereby obtaining at least one engineered protein binding domain candidate;
iii) pre-screening of the at least one engineered protein binding domain candidate, wherein the pre-screening comprises analyzing an alignment of the at least one engineered protein binding domain candidate to the target protein, and/or the binding of the at least one engineered protein binding domain candidate to the target protein;
iv) optionally, calculating the binding probability of the engineered protein binding domain candidate to the target protein;
v) selecting at least one engineered protein binding domain, wherein an engineered protein binding domain candidate with good alignment or binding to the target protein is classified as an engineered protein binding domain;
vi) generating at least one expression vector containing a nucleotide sequence of a CAR with one of the engineered protein binding domains; wherein, preferably, each expression vector contains a different nucleotide sequence of a CAR molecule and/or the engineered protein binding domain; and/or
vii) generating at least one expression vector containing a nucleotide sequence of a bispecific molecule with one of the engineered protein binding domains; wherein, preferably, each expression vector contains a different nucleotide sequence of a bispecific molecule and/or the engineered protein binding domain;
viii) transfecting one expression vector of step vi) and/or step vii) in an immune effector cell, such as a T cell, resulting in a CAR expressing immune effector cell product, comprising the engineered protein binding domain; or transfecting one expression vector of step vii) in a cell, such as a HEK293 cell, a HEK293T cell, a CHO cell, a HT-1080 cell, or a PER.C6 cell, resulting in a cell producing the bispecific molecule comprising the engineered protein binding domain; and
ix) optionally, isolating said bispecific molecule comprising the engineered protein binding domain;
thereby obtaining the CAR expressing immune effector cell product, such as the CAR-T cell product, and/or the HIT expressing immune effector cell product, such as the HIT-T cell product, and/or the bispecific molecule expressing immune effector cell product, such as the BiTE expressing immune effector cell product, comprising the immunomodulatory molecule, and/or the bispecific molecule, such as the BiTE, comprising the engineered protein binding domain.

14. The method according to claim 13, wherein the target region is a flat, plain, and/or hydrophobic target region; and/or wherein the target region comprises at least one beta sheet; and/or wherein the target region is an accessible target region and/or is not buried in a cell membrane; and/or wherein the target region does not comprise a salt bridge;
wherein, optionally, the method further comprises the step of:
x) functionally testing the CAR expressing immune effector cell product, such as the CAR-T cell product, the HIT expressing immune effector cell product, such as the HIT-T cell product, the bispecific molecule expressing immune effector cell product, such as the BiTE expressing immune effector cell product, comprising the immunomodulatory molecule, and/or the bispecific molecule, such as the BiTE, comprising the engineered protein binding domain;
wherein, further optionally, the functionally testing in step x) of the method comprises measuring and/or analyzing a T cell signaling molecule, such as a luminescence-based readout of a nuclear factor of activated T cells (NFAT) activation and/or a cytokine or lytic protein produced by a T cell and/or the proliferation of a T cell in response to the targeted antigen and/or the death of cancer cells.

15. A product obtainable by a method according to claim 13 or 14.

16. A pharmaceutical composition comprising the immunomodulatory molecule according to any of the claims 1 to 10, the engineered protein binding domain according to claim 11, the isolated nucleic acid of claim 12, or the product of claim 15, wherein, optionally, said pharmaceutical composition comprises one or more pharmaceutically acceptable excipient(s) and/or carrier(s); wherein, further optionally, said pharmaceutical composition comprises one or more further agent(s) or drug(s), such as chemotherapeutic drugs, radioisotopes, antibody-drug conjugates, immune checkpoint inhibitors, bispecific molecules, such as BiTEs, BiTE-secreting cells, and/or CAR-T cells.

17. The immunomodulatory molecule according to any of the claims 1 to 10, the engineered protein binding domain according to claim 11, the isolated nucleic acid of claim 12, the product of claim 15, or the pharmaceutical composition of claim 16, for use in a method of diagnosis, prevention and/or treatment of a disease, or for preventing an organ transplant rejection, in a patient in need thereof; wherein, preferably, said patient is a mammal; wherein, more preferably, said patient is a human;
wherein, optionally, the disease is selected from cancer, autoimmune diseases, infectious diseases, cardiovascular diseases, cerebrovascular diseases, neurodegenerative diseases, dermatological diseases, genetic diseases, aging-related diseases, nephrological diseases, endocrinological diseases, fibrotic diseases, metabolic diseases, obesity-related diseases, gynecological diseases, ophthalmic diseases, otorhinolaryngologic diseases, allergies, and an immune deficiency;
wherein, further optionally, the cancer is selected from one or more of brain tumor, esophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancers, skin cancer, leukemia, lymphoma, myeloma, plasma cell leukemia, plasma cell dyscrasia, myeloid malignancies, lymphoid malignancies, breast cancer, ovarian cancer, neuroendocrine carcinoma, endometrial cancer, vaginal cancers, blood-related cancers, uterine cancer, testicular cancer, glioma, bone sarcoma, cervix cancer, synovial sarcoma, and neuroendocrine carcinoma, for example high-grade poorly differentiated neuroendocrine carcinoma (NEC-G3); wherein, preferably, the cancer is selected from lung cancer, pancreatic cancer, colon cancer, rectal cancer, ovarian cancer, myeloma, plasma cell leukemia, and plasma cell dyscrasia; more preferably lung cancer, pancreatic cancer, colon cancer, rectal cancer, myeloma, and plasma cell leukemia; most preferably lung cancer, and myeloma;
wherein, further optionally, the cancer is a metastatic, stage III cancer, or stage IV cancer.

18. The immunomodulatory molecule according to any of the claims 1 to 10, the engineered protein binding domain according to claim 11, the isolated nucleic acid of claim 12, the product of claim 13, or the pharmaceutical composition of claim 14, for use in a method according to claim 17, wherein the immunomodulatory molecule is delivered to target cells in vitro, ex vivo, and/or in vivo, optionally wherein the delivery of the immunomodulatory molecule generates a cell therapy and/or is used as a cell therapy, wherein:
(a) the immunomodulatory molecule is delivered via a lentivirus, an adeno-associated virus (AAV), a lipid nanoparticle (LNP), a viral-like particle (VLP), or electroporation, wherein the immunomodulatory molecule is preferably delivered in form of a nucleic acid, such as a DNA or mRNA, or in form of a protein, optionally wherein the immunomodulatory molecule is permanently or transiently expressed in the target cells after delivery, and/or
(b) a nucleic acid donor template, such as a DNA donor template, containing the immunomodulatory molecule, a gRNA using homology-directed repair (HDR) gene editing, and a lentivirus, a transposase, such as a Sleeping Beauty or a piggyBac transposase, or a CRISPR-nuclease, is delivered to said target cells, and wherein the immunomodulatory molecule is semi-randomly integrated into a target cell via the lentivirus, the transposase, such as the Sleeping Beauty or the piggyBac transposase, or the CRISPR-nuclease, and/or
(c) the immunomodulatory molecule is delivered via a CRISPR-mediated HDR or a prime editing and/or recombinase based approach, such as a PASTE or a PASSIGE based approach, wherein the immunomodulatory molecule is targetedly integrated into a target cell via the CRISPR-mediated HDR or the prime editing and/or recombinase based approach, such as the PASTE or the PASSIGE based approach, wherein, optionally, the immunomodulatory molecule is integrated targetedly at the locus of the TRAC gene or at the locus of a safe harbor gene.
